(19) Europäisches Patentamt / European Patent Office / Office européen des brevets

(11) **EP 2 582 334 B1**

(12) **EUROPEAN PATENT SPECIFICATION**

(45) Date of publication and mention
of the grant of the patent:
**16.12.2015 Bulletin 2015/51**

(21) Application number: **11726633.8**

(22) Date of filing: **14.06.2011**

(51) Int Cl.:
***A61F 5/00*** (2006.01)

(86) International application number:
**PCT/US2011/040257**

(87) International publication number:
**WO 2011/159643 (22.12.2011 Gazette 2011/51)**

(54) **HYDRAULIC-MECHANICAL GASTRIC BAND**

HYDRAULISCH-MECHANISCHES MAGENBAND

ANNEAU GASTRIQUE HYDRAULIQUE-MÉCANIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2010 US 816310**

(43) Date of publication of application:
**24.04.2013 Bulletin 2013/17**

(73) Proprietor: **APOLLO ENDOSURGERY, INC.
Austin, TX 78746 (US)**

(72) Inventors:
• **BIRK, Janel
Oxnard, California 93036 (US)**

• **FRIDEZ, Pierre
1055 Froideville (CH)**
• **BERTOLOTE, Tiago
1202 Geneva (CH)**

(74) Representative: **Hoffmann Eitle
Patent- und Rechtsanwälte PartmbB
Arabellastraße 30
81925 München (DE)**

(56) References cited:
**EP-A2- 1 719 480        US-A- 5 938 669
US-A1- 2007 156 013     US-A1- 2009 054 914
US-A1- 2009 270 904**

## Description

### FIELD

This invention relates to surgical devices for regulating or controlling an organ or a duct, for example, a gastric banding system.

### BACKGROUND

Obesity is well recognized as a serious health problem, and is associated with numerous health complications, ranging from non-fatal conditions to life threatening chronic diseases. According to the World Health Organization, debilitating health problems associated with obesity include respiratory difficulties, chronic musculoskeletal problems, skin problems and infertility. Life-threatening problems fall into four main areas: cardiovascular disease problems; conditions associated with insulin resistance such as type 2 diabetes; certain types of cancers, especially the hormonally related and large bowel cancers; and gallbladder disease. Beyond these physiological problems, obesity has also psychological consequences, ranging from lowered self-esteem to clinical depression.

Surgical intervention is sometimes indicated for people suffering from the effects of obesity. Such intervention not only mitigates the myriad health problems arising from being overweight, but may reduce the risk of early death of the patient. Left untreated, morbid obesity may reduce a patient's life expectancy by ten to fifteen years.

Documents cited during prosecution include: US5938669A; EP1719480 A2; US2009/270904 A1; US2007/156013 A1; and US2009054914 A1.

### SUMMARY

An implantable banding system for treating obesity is disclosed. The implantable banding system includes a telemetric control unit, a gastric band having at least one inner fluid compartment and an outer mechanical adjustment mechanism, the at least one inner fluid compartment being filled with a fixed volume of fluid, and the outer mechanical adjustment mechanism comprising a device configured to adjust the gastric band through a variety of diameters, an implant circuit coupled to the device and configured to receive an adjustment signal to control the operations of the device, and a sensor positioned within the at least one inner fluid compartment, configured to monitor a parameter of the fixed volume of fluid, generate an adjustment signal based on the parameter and one or more parameter control limits, and automatically activate the device based on the adjustment signal or transmit the adjustment signal to the telemetric control unit.

A system for regulating an organ or duct, for example, the functioning of an organ or duct, is provided.

The system generally comprises an implantable band having a first end and a second end, a distal region and a proximal region, and a connector configured to couple the first end with the second end such that the band is formable into a loop configuration. The band is structured to circumscribe, or at least partially circumscribe, an organ or duct, for example, a stomach. The system further comprises a mechanism for enabling adjustment of an inner circumference of the loop configuration to effect constriction of the organ or duct.

For the sake of simplicity, and in no way intended to limit the scope of the invention, the "organ or duct" will hereinafter typically be referred to as a "stomach" and the system will be described as a gastric band system. The band is structured to circumscribe an upper portion of a stomach to form a stoma that controls the intake of food to the stomach. It is to be appreciated that although the invention is hereinafter typically described as pertaining to a gastric band system for application to a stomach, for example, for obesity treatment, the system, with appropriate modification thereto, can be used for regulating or controlling any organ or duct that would benefit from application of the present system thereto.

Once the band is implanted about the stomach, the size of an inner diameter of the band can be adjusted to provide the desired degree of restriction. Techniques for determining appropriate adjustment of gastric bands, timing and amount of adjustments, are known in the art and therefore will not be described in great detail herein.

Advantageously, in a broad aspect of the invention, the system may be structured to substantially prevent or at least reduce the occurrence of pinching of the body tissues, for example, the tissues of the stomach, during constriction or tightening of the band.

For example, in a specific embodiment, the system further comprises a contact region located between the first end and the second end of the band which is structured and functions to progressively move tissue, for example stomach tissue, during tightening of the band, without entrapping the tissue.

The contact region may comprise a plurality of first segments and a plurality of second segments arranged in a generally alternating manner along the proximal (e.g. stomach-facing) region of the band. The first segments may comprise relatively wide, substantially incompressible cushion segments, and the second segments may comprise relatively thin, elastic tension segments. During constriction of the band, adjacent incompressible cushion segments form a progressively narrowing angle, for example, a substantially V-shaped surface. A tension segment is located between the adjacent cushion segments and forms the vertex of the angle or V.

In some embodiments, the cushion segments and tension segments form an inner circumference of the loop configuration having a generally star-shape, defined by the contact region. Deformation of the star-shape during adjustment substantially or entirely prevents pinching of tissues, as the cushion segments roll forward towards one another without gaps there-between thus pushing the tissue inwardly.

More specifically, in some embodiments, the contact region defines alternating convex stomach-facing surfaces and concave stomach-facing surfaces. The convex organ facing surfaces may be defined by the cushion segments and the convex organ facing surfaces are defined by the tension segments located between adjacent cushion segments. During constriction of the band, the convex organ-facing surfaces may maintain their shape while folding at the tension segments inwardly toward one another. This mechanism and structure causes the tissues of the stomach to be pushed outwardly from the band constriction without the tissues becoming entrapped and/or pinched by the contact region.

In addition, the structure of the contact region, including cushion segments and tension segments, may be advantageously structured to maintain mechanical stability of the band. For example, the tension segments provide a means for maintaining positioning of the cushion segments and by substantially preventing the contact region of the band from creasing, folding or rolling out of position while the band is implanted in the body around the duct or organ, for example, the stomach.

In some embodiments, the contact region comprises a membrane, for example, a somewhat tubular-shaped elastic membrane encompassing, secured to or defining the cushion segments. In one embodiment, portions of the membrane may form the tension segments between adjacent cushion segments.

In one embodiment, the cushion segments are formed of individual incompressible molded elements in contact with or spaced apart from one another, and affixed to the membrane. The cushion segments may be spaced apart by portions of the elastic membrane which are stretched under tension.

The cushion segments may be located on an internal surface of the membrane or alternatively may be located on an external surface of the membrane. In one embodiment, the cushion segments are located on an external surface of the membrane and are overmolded to the membrane.

In another feature of the invention, the membrane may include structure, for example, corrugations or indentations, for facilitating expansion of the membrane during adjustment of the loop. For example, such corrugations can be located and structured to minimize the force required to elongate or stretch the membrane in the radial direction during tightening of the band. The corrugated surfaces of the membrane reduce membrane deformation energy by allowing the membrane to unfold rather than stretch during adjustment.

The mechanism for enabling adjustment may comprise an electronic interface, for example, an implantable electronic interface connected to the band, and a control, for example an external control unit, capable of communicating with the interface to regulate the constriction of the band about the organ or the duct.

These and other features of the present invention may be more clearly understood and appreciated upon consideration of the following Detailed Description and the accompanying Drawings.

**BRIEF DESCRIPTION OF THE DRAWINGS**

FIG. 1 shows a system including a band having a contact region, an interface having an antenna/controller pod, and an external control in accordance with an exemplary embodiment of the present invention.

FIG. 2 shows a perspective, cutaway view of the contact region shown in FIG. 1 in accordance with an exemplary embodiment of the present invention.

FIG. 3 shows a perspective view of the contact region shown in FIG. 1 in accordance with an exemplary embodiment of the present invention.

FIG. 3A shows a cross-sectional view of the contact region taken along lines 3A-3A of FIG. 3 in accordance with an exemplary embodiment of the present invention.

FIG. 4A shows an elevation view of the contact region shown in FIG. 1 in accordance with an exemplary embodiment of the present invention.

FIG. 4B shows an elevation view of an alternative contact region in accordance with an exemplary embodiment of the present invention.

FIG. 4C shows a perspective view of the alternative contact region shown in FIG. 4B in accordance with an exemplary embodiment of the present invention.

FIG. 5A shows a cross-sectional view of the band shown in FIG. 1 in accordance with an exemplary embodiment of the present invention.

FIG. 5B shows a cross-sectional view of the band taken along lines 5B-5B of FIG. 5A in accordance with an exemplary embodiment of the present invention.

FIG. 5C shows a perspective, cutaway view of the band in a fully open position in accordance with an exemplary embodiment of the present invention.

FIG. 5D shows a perspective, cutaway view of the band in a constricted position in accordance with an exemplary embodiment of the present invention.

FIGS. 5E and 5F are schematic representations of an amplified adjustment feature in accordance with an exemplary embodiment of the present invention.

FIGS. 5G and 5H are simplified schematic representations of another embodiment of the invention in accordance with an exemplary embodiment of the present invention.

FIGS. 6A through 6C show plan views of the band at different levels of constriction in accordance with an exemplary embodiment of the present invention.

FIG. 7 is a partial perspective view of a screw thread portion of a tension element useful in the band of the system in accordance with an exemplary embodiment of the present invention.

FIG. 8 is a perspective view of the entire tension element shown in FIG. 7 in accordance with an exemplary embodiment of the present invention.

FIG. 9 is a perspective view of the entire tension element of FIG. 8 coupled to a rigid distal peripheral portion in the band of the system in accordance with an exemplary embodiment of the present invention.

FIG. 10 is a perspective view of the band of the system in a straightened configuration and located within a trocar to facilitate implantation in accordance with an exemplary embodiment of the present invention.

FIG. 11 is a cross-sectional view of an actuator housing on an end of the band in accordance with an exemplary embodiment of the present invention.

FIG. 12 is a perspective view of an actuator in the housing shown in FIG. 11 in accordance with an exemplary embodiment of the present invention.

FIG. 13 is a perspective view of the tension element engaged with the actuator shown in FIG. 12 in accordance with an exemplary embodiment of the present invention.

FIG. 14 is a cross-sectional view depicting the construction of the actuator shown in FIG. 12 in accordance with an exemplary embodiment of the present invention.

FIG. 15 is a cross-sectional view depicting the construction of a reference position switch useful in the system in accordance with an exemplary embodiment of the present invention.

FIGS. 16A and 16B are perspective views illustrating a clip used to close the band of the system in accordance with an exemplary embodiment of the present invention.

FIG. 17 is a perspective view of the antennae/controller pod of the system shown in FIG. 1 in accordance with an exemplary embodiment of the present invention.

FIG. 18 is a cut-away view of the interior of the implantable antenna/controller pod in accordance with an exemplary embodiment of the present invention.

FIG. 19 is a schematic view of telemetric power and control circuitry useful in the system in accordance with an exemplary embodiment of the present invention.

FIG. 20 is a view of a signal strength indicator portion of the control shown in FIG. 1 in accordance with an exemplary embodiment of the present invention.

FIG. 21 is a schematic diagram illustrating placement of the implantable portion of the system in accordance with an exemplary embodiment of the present invention.

FIGS. 22A-22H are each a view illustrating steps in a method of laparoscopically implanting the system in accordance with an exemplary embodiment of the present invention.

FIG. 23 is a perspective view of a contact region including a membrane and overmolded incompressible cushions of a gastric band in accordance with an exemplary embodiment of the present invention.

FIGS. 24 and 25 are cross-sectional views of the contact region shown in FIG. 23 taken along line 24-24 and line 25-25, respectively, in accordance with an exemplary embodiment of the present invention.

FIGS. 26 and 26A show dilated cushion segments and tension segments forming an inner circumference of the loop configuration having a generally star-shape in accordance with an exemplary embodiment of the present invention.

FIGS. 27 and 27A show constricted cushion segments and tension segments forming an inner circumference of the loop configuration having a generally star-shape in accordance with an exemplary embodiment of the present invention.

FIG. 28 illustrates an implantable banding system in accordance with an exemplary embodiment of the present invention.

FIGS. 29A and 29B illustrate exemplary fixed-volume compartments in accordance with an exemplary embodiment of the present invention.

FIG. 30 illustrates an implantable banding system further comprising an override mechanism in accordance with an exemplary embodiment of the present invention.

FIG. 31 illustrates a working embodiment of an implantable banding system in accordance with an exemplary embodiment of the present invention.

FIG. 32 illustrates a pressure chart according to an embodiment of the present invention.

FIG. 33 depicts a sectional view of a gastric band according to an embodiment of the present invention.

FIG. 34 depicts a sectional view of a gastric band according to an embodiment of the present invention.

## DETAILED DESCRIPTION

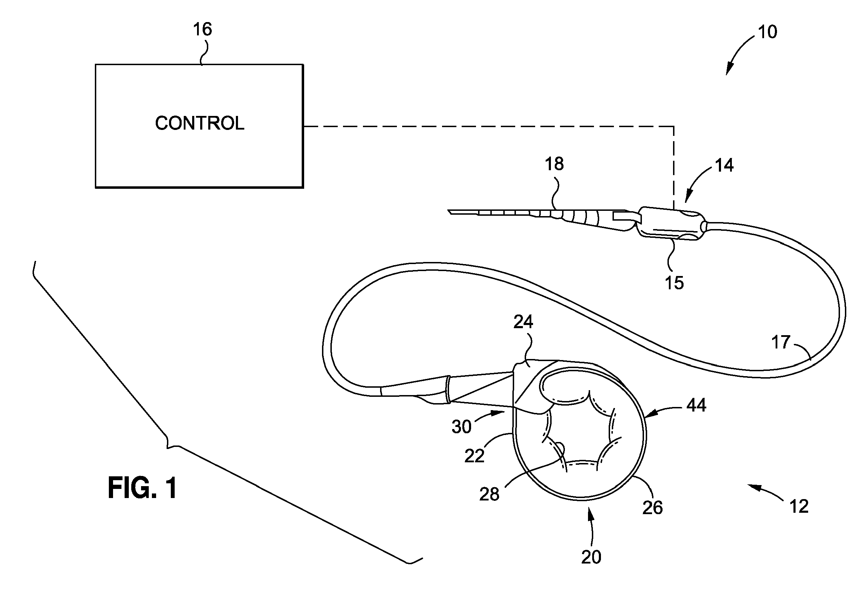

Turning now to FIG. 1, an embodiment of a system of the present invention is generally shown at **10.** In one embodiment of the present invention, the system **10** is useful for regulating the functioning of an organ or duct (not shown), for example, a stomach or a stoma of the stomach. In one embodiment, the system **10** is a gastric banding system useful in the treatment of obesity and/or obesity related diseases.

It is to be understood that although much of the following description is generally directed to gastric banding systems of the invention, the present invention is in no way limited thereto. Other embodiments of the invention may be applied to regulate the functioning of other body organs or ducts, such as in the treatment of gastroesophageal reflux disease, urinary or fecal incontinence, colostomy, or to regulate blood flow.

In this exemplary embodiment, the system **10** generally comprises an implantable portion **12** including an adjustable band **20,** an interface **14** including an antenna/controller pod **15,** and a control **16** in communication, for example, telemetric communication, with the pod **15.** The pod **15** may be connected to the band **20** by means of an antenna cable **17** and may include a removable tab **18** for facilitating laparoscopic positioning thereof.

Laparoscopically implanted gastric bands and their use in the treatment of obesity are now well known. Generally, in accordance with the present invention, the band **20** is structured to be implantable in a patient, for example, laparoscopically implantable, around an upper region of the patient's stomach, thereby forming a stoma that restricts food intake and provides feelings of satiety. The inner diameter of the band **20** is adjustable in vivo in order to enable a physician or patient to achieve most desirable stoma size, and the best clinical results.

The band **20** includes a first end **22** and a second end **24,** a distal region **26** and a proximal region **28,** and a connector **30** configured to couple the first end **22** with the second end **24** of the band **20** such that the band **20** is formable into a loop configuration, as shown.

When the band **20** is formed into the loop configuration, the proximal region **28** forms an inner circumferential surface which at least partially circumscribes and contacts the organ or duct, for example, the stomach portion, to be regulated or controlled.

Generally, by loosening or tightening the band **20** about the stomach portion, regulation and/or functioning of the stomach can be controlled or adjusted. When not connected at the first and second ends **22, 24,** the band **20** can be temporarily straightened in order to facilitate surgical implantation, for example, via laparoscopic techniques.

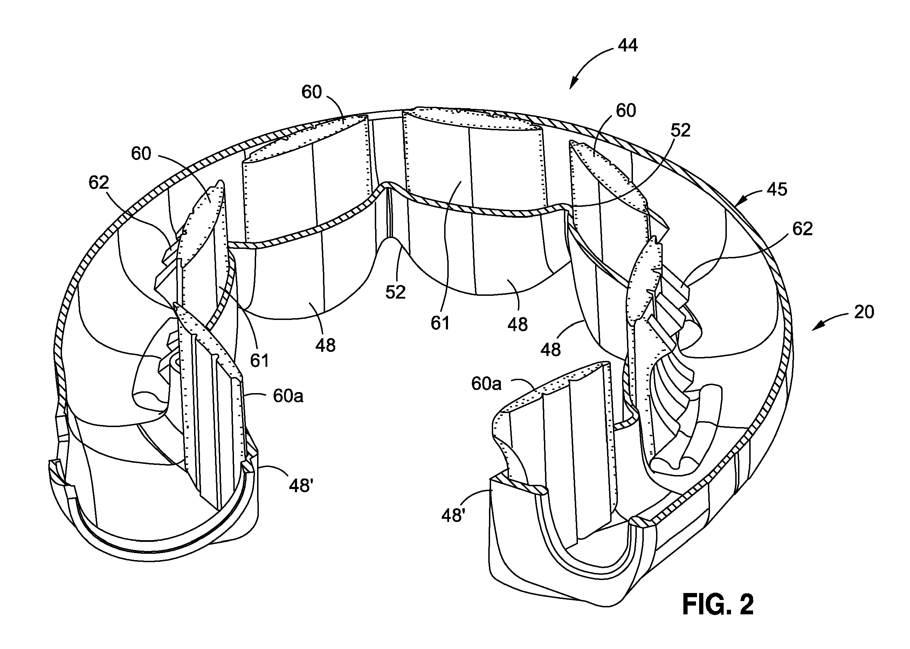

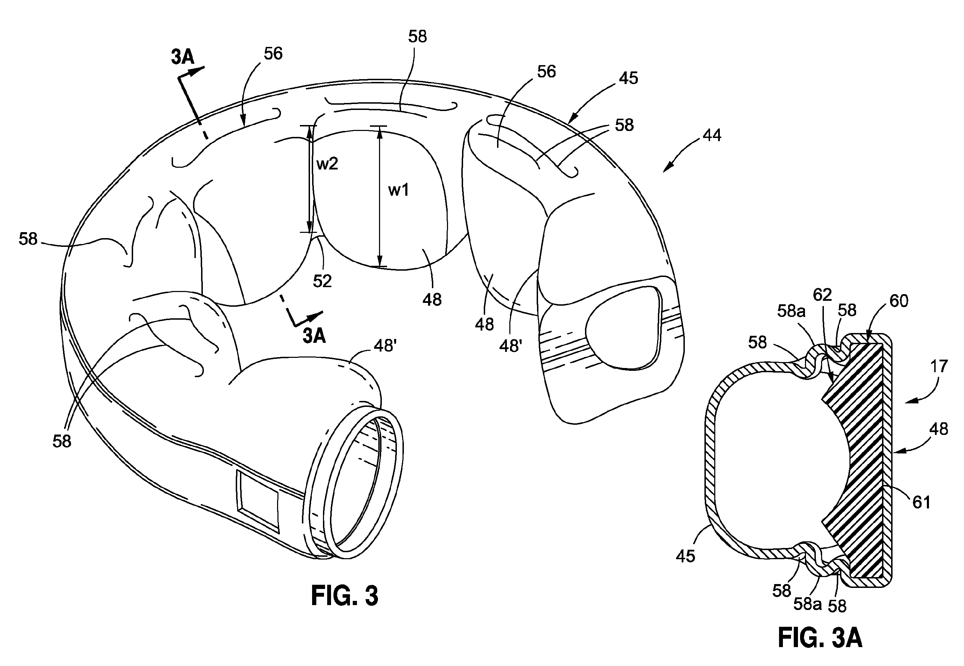

The system **10** further comprises a contact region **44** disposed between the first and the second ends **22, 24** of the band **20.** Turning now to FIGS. 2 and 3, the contact region **44** may comprise, at least in part, an elastic component made of, for example, a molded silicone elastomer. The elastic component comprises a membrane **45** having a generally tubular form which covers or encases the internal mechanisms of the band **20,** for example, the gastric band tightening mechanisms such as those to be described hereinafter. The membrane **45,** when at rest, may have an arcuate or C-shaped form.

As shown in FIG. 2, the contact region **44** comprises first segments **48** and second segments **52** arranged in a generally alternating manner. The first segments **48** may be defined by generally planar and/or convex stomach-facing surfaces, i.e., proximal surfaces, of the contact region **44.** The second segments **52** may be defined by generally concave exterior surfaces generally forming indentations between the first segments **48.**

In some embodiments, the first segments **48** comprise cushions **60.** The cushions **60** are spaced apart from one another by the second segments **52.** The cushions **60** may be made of noncompressible material, for example, a silicone elastomer.

In one embodiment of the present invention, a suitable incompressible material making up the cushions **60** is a moldable material that has substantially constant density throughout and maintains its volume when deformed. The volume of incompressible materials cannot be reduced more than a nominal amount (e.g., about 5%) when subjected to static compression, or external pressure. The cushions **60** may be a soft silicone material that is a deformable, resilient solid or a gel. In other embodiments, the cushions **60** may be filled with noncompressible liquid, for example, a saline solution.

The cushions **60** may be made of a material that has a different durometer, for example, is softer than the material forming the membrane **45.** In a specific embodiment, the cushions **60** comprise a soft, molded silicone elastomer material having a hardness of about 5 Shore A. The membrane **45** comprises a soft molded silicone elastomer material having a hardness of about 30 Shore A.

In one embodiment, the cushions **60** may be structured to provide form, definition, support and/or structural integrity to the first segments **48**. The second segments **52** may be portions of the membrane **45** which are stretched under tension. The second segments **52** may be structured to provide stability to the contact region **44** and to maintain positioning, for example, circumferential positioning, of the cushions **60** during use of the system **10.**

Turning now to FIG. 3, the first segments **48** may have a first axial width W1, and the second segments **52** may have a second axial width W2 which is less than the first axial width W1.

In the shown embodiment of the present invention, the contact region **44** includes seven first segments **48** (including **48'**), each first segment being generally equally spaced apart by intermediate second segments **52**. In other embodiments of the present invention, the contact region **44** includes at least three first segments, at least four first segments, at least five first segments, or at least six first segments. In other embodiments of the present invention, the contact region **44** includes more than seven first segments, for example, up to ten first segments or more.

In another embodiment of the present invention, the membrane **45** may be structured to facilitate expansion in a radial direction during adjustment of the inner circumference of the band **20**. For example, turning now to FIG. 3, the membrane **45** may include radially expandable surfaces **56**. For example, the membrane **45** includes one or more corrugations **58.**

In the shown embodiment, the corrugations **58** are generally aligned with the cushions **60**. As shown in FIG. 3A, the corrugations **58** may be defined by convolutions **58a** defined in an upper surface and/or a lower surface of the membrane **45**. The corrugations **58** may be placed to minimize the force required by the actuating mechanism to elongate the membrane **45** in the radial direction. Rather than requiring excessive stretching of the membrane **45,** the membrane **45** unfolds during adjustment.

In the shown embodiment, certain first segments **48** include corrugations **58** and other first segments (e.g. first segments **48'**) do not include corrugations. For example, intermediate first segments **48** include corrugations **58** and terminal first segments **48'** do not include corrugations.

The presently described and shown corrugated structure of the contact region **44** may function to facilitate controlled expansion and/or contraction of the first segments **48,** for example, during adjustment of the inner circumference of the band. In some embodiments of the invention, the corrugated surfaces **56** function, at least in part, to decrease the level of force required to adjust the inner circumference of the loop.

In some embodiments, the contact region **44** includes first cushions **60** and second cushions **60a** which are configured somewhat differently than first cushions **60** (see FIG. 2). In the shown embodiment, first cushions **60** are located on intermediate first segments **48** and second cushions **60a** are located on terminal first segments **48'** (i.e., those first segments located at the extremities of the contact region **44**).

More specifically, in the embodiment shown in FIG. 2, each first cushion **60** includes a substantially planar or convex face **61** and at least one or more distal projections **62**. For example, each cushion **60** includes three longitudinal, arcuate projections **62** as shown. A cross-sectional view of first cushion **60** having these features is also shown in FIG. 3A.

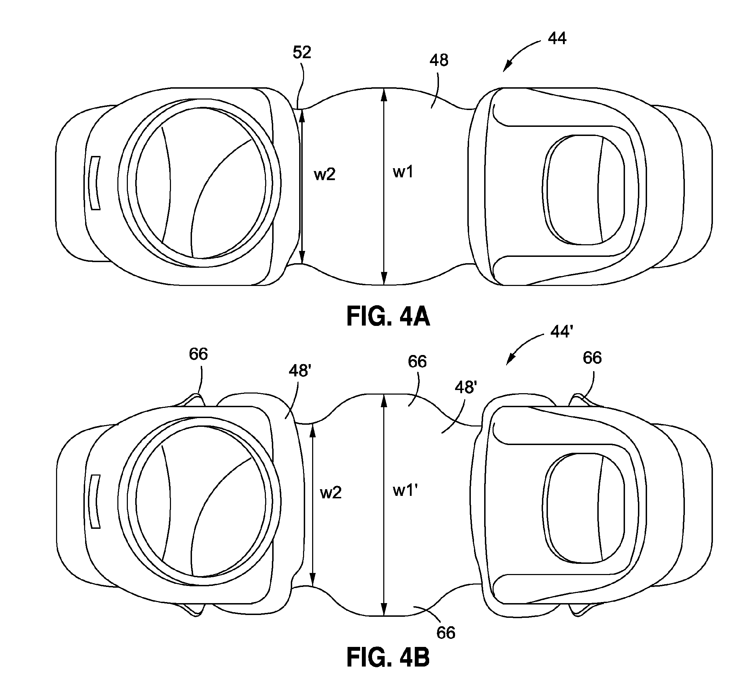

FIG. 4A shows an elevation view of the contact region **44** (cushions not shown) in order to illustrate width W1 of first segment **48** relative to width W2 of second segment **52** of contact region **44**. In an exemplary embodiment of the present invention, W1 is about 17 mm and W2 is about 13 mm.

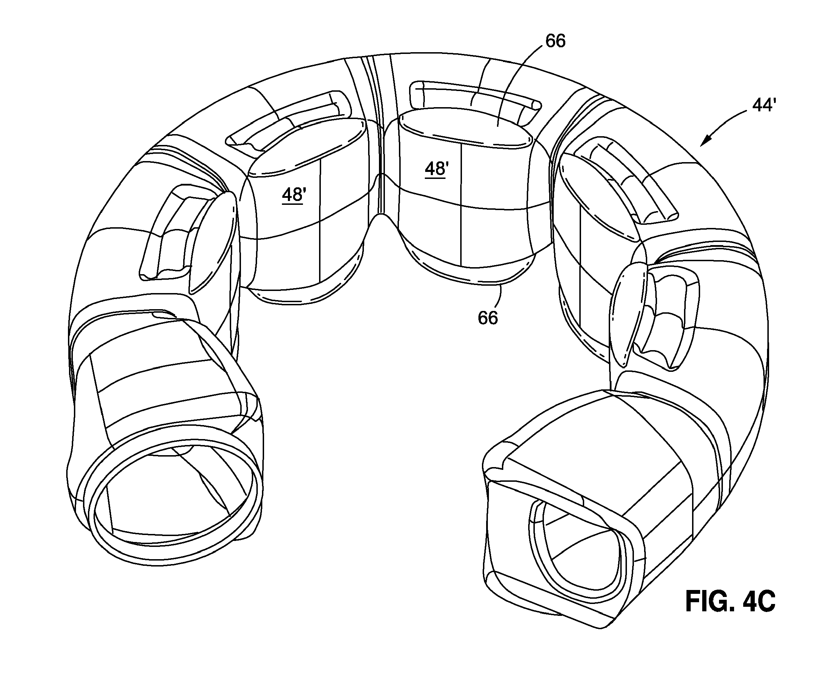

FIG. 4B shows an elevation view of an alternative contact region **44'** in accordance with an exemplary embodiment of the present invention. The contact region **44'** is identical to the contact region **44** shown in FIG. 4A, with a primary difference being that the first segment width W1' of contact region **44'** is greater than first segment width W1 of contact region **44**. That is, W1' > W1. The additional width of the first segment width W1' is provided by upper and lower protuberances **66** on the first segments **48'**. In an exemplary embodiment, W1' is about 19 mm and W2 is about 13 mm. FIG. 4C shows a perspective view of the contact region **44'** having first segments **48'** with protuberances **66.**

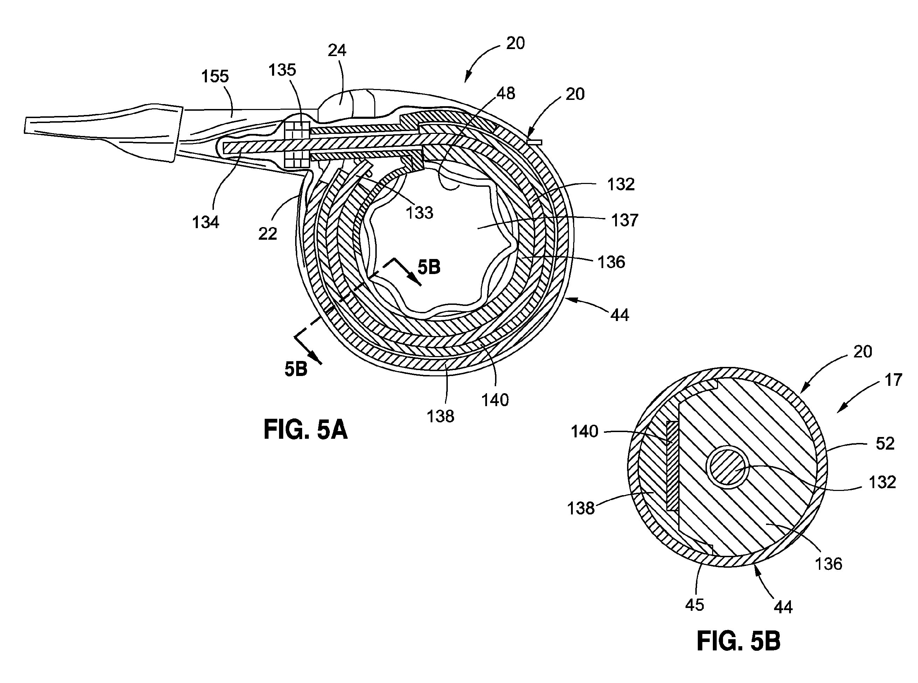

Turning now to FIGS. 5A-5D, an exemplary inner mechanism of the band **20** which enables adjustment of the inner circumference of the loop configuration will now be described. The band **20** may comprise a flexible tension element **132** having a fixed end **133** mounted to a first end **22** of the band **20** and another end **134** that is coupled to an actuator **135** at a second end **24** of the band **20**. The flexible tension element **132** is slidingly disposed within a substantially cylindrical tube of axially compressible material **136**. When the flexible tension element **132** is pulled through the actuator **135,** a compressible material **136** is compressed and the diameter of loop opening **137** is reduced.

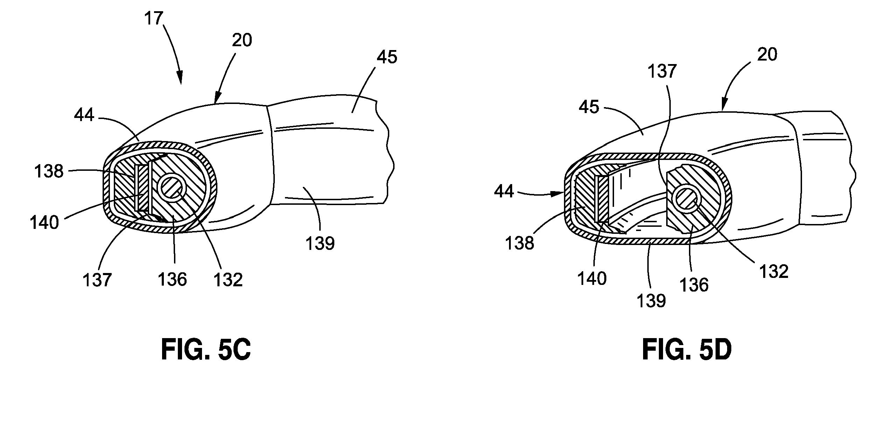

Turning now specifically to FIGS. 5C and 5D, a compressible material **136** may be surrounded on a distal face **137** thereof with a flexible, relatively sturdy elastomeric material, such as a silicone element **138**. Both the compressible material **136** and the silicone element **138** are enclosed within the membrane **45** of the contact region **44.**

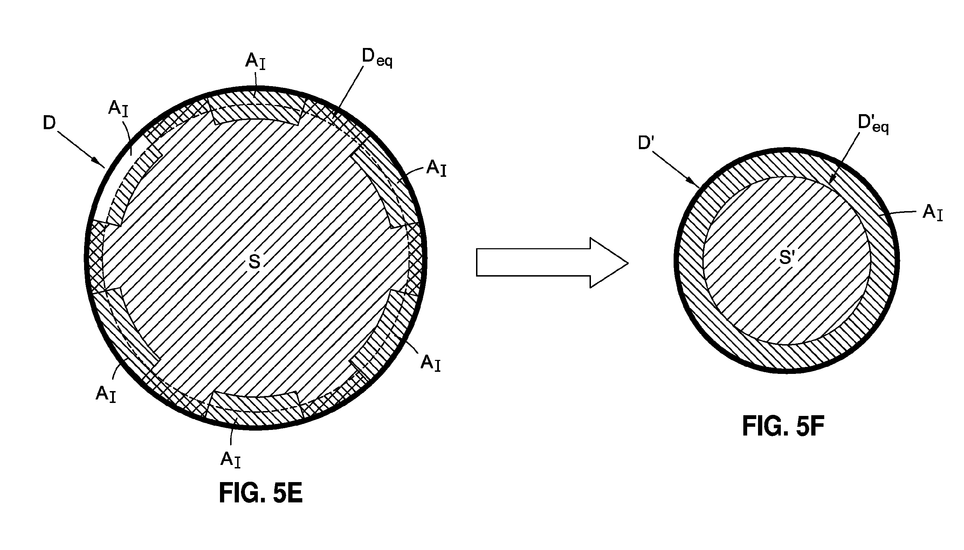

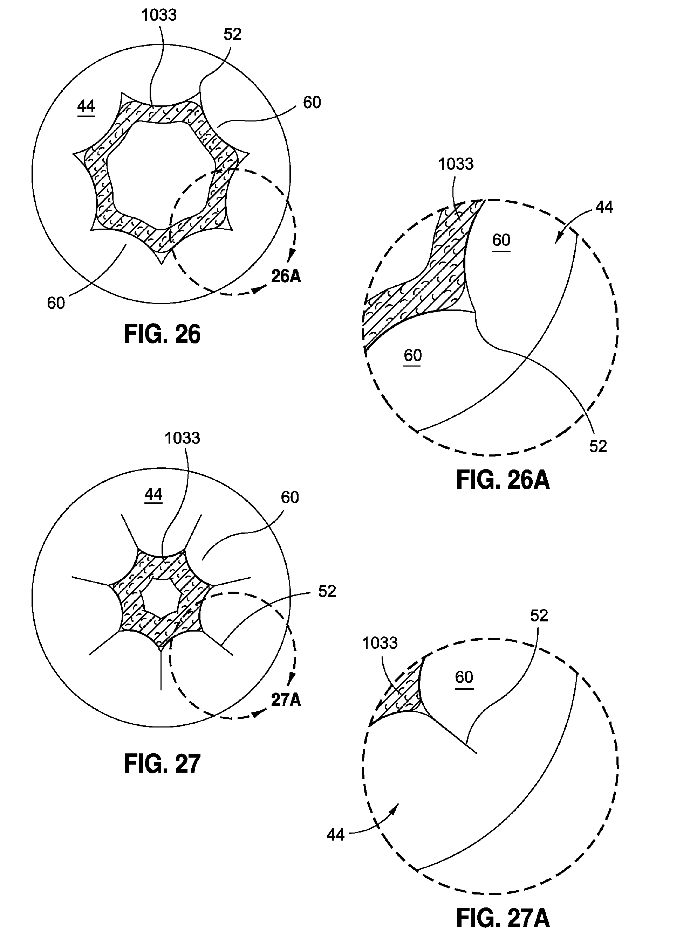

In one embodiment of the present invention, the band **20** may be structured to provide an amplified adjustment feature. This concept is illustrated in FIGS. 5E and 5F and in FIGS. 26 thru 27A.

The incompressible cushions **60** provide enhanced and more efficient control of adjustment of the inner diameter of the band **20**. FIGS. 5E and 5F are schematic representations of the cross-section of the band **20** in the open configuration and constricted configuration, respectively. The outer diameter D represents the outer diameter of an axially adjustable portion of the band **20**. Areas of the individual cushions **60** are represented by areas $A_I$ in FIG. 5E (open configuration). The total area occupied by the individual cushions is represented as annular area $A_T$ in FIG. 5F (constricted configuration). The surface S represents the available lumen around the stomach (or other organ or duct being controlled or regulated) and diameter Deq represents an equivalent diameter, that is, the diameter of a circle having the same surface area as S.

When the loop is constricted from the fully open state, diameter D (FIG. 5E) becomes D' (FIG. 5F), the surface S becomes S' and the equivalent diameter Deq becomes D'eq. Because the cushions occupying $A_I$ are incompressible, the total surface area $A_T$ occupied by the cushions does not change. The equivalent diameter Deq decreases more rapidly than the diameter D.

For example, D = 29 mm in a fully open position and a total surface of the incompressible cushions $A_T$ equal to about 120 square mm: S = 540.52 sq mm and Deq = 26.2 mm. When in the fully closed position, D' = 19 mm: S' = 163.53 sq mm, and D'eq = 14.4. Thus D-D' = 10 mm, and Deq-D'eq = 11.8 mm, which provides an "amplification factor" of about 1.18. Thus, by changing the values of D, D' and $A_T$, the amplification factor can be controlled.

The substantially incompressible cushion segments allow a relative restriction of the lumen during adjustment greater than without substantially incompressible cushion segments. That greater relative restriction arises from the fact that the cross-section of the substantially incompressible cushion segments remains constant during adjustment, whereas the area of the lumen decreases during closure, so that the ratio (cushion cross-section)/(lumen) increases. Accordingly, the substantially incompressible cushion segment effect on lumen restriction increases during closure.

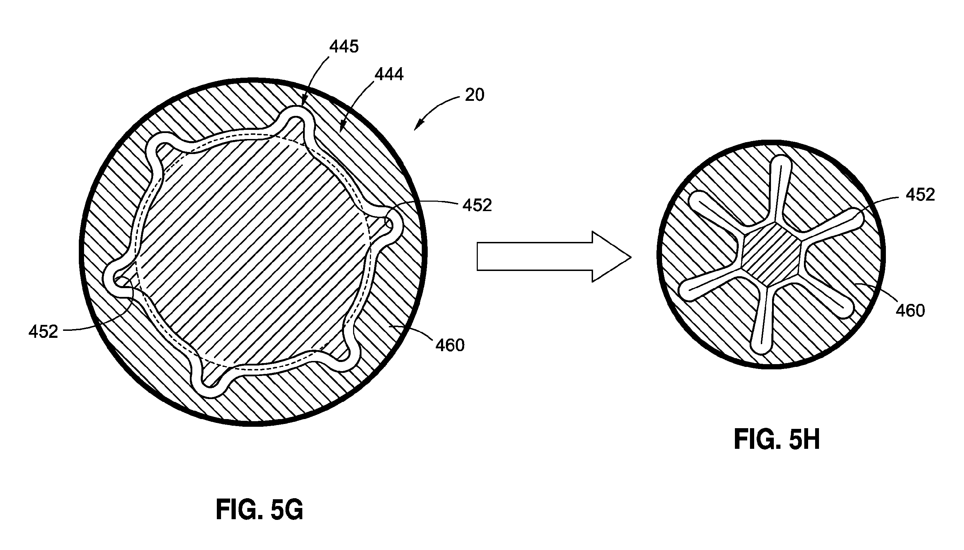

FIGS. 5G and 5H show a simplified schematic representation in which the contact region **444** comprises an elastic membrane **445** and a single continuous, incompressible cushion segment **460** instead of the individual, separate cushion segments **60** shown in FIG. 2. Other than cushion segment **460** being a single substantially continuous cushion segment rather than a plurality of individual separate cushion segments **60**, the band **20** (FIG. 5G) may be identical to the band **20** (FIG. 2). The continuous cushion segment **460** is configured or shaped to accommodate tension segments **452** of the membrane **445**. For example, the continuous cushion segment **460** has a variable thickness, with the thickest regions functioning similarly to incompressible cushion regions **60** described elsewhere herein. FIG. 5H shows bending of tension regions **452** and deformation of incompressible cushions **460** during the constriction of the loop.

Turning back to FIG. 5A, the band **20** may further comprise member **140** of a relatively rigid material. By its structural rigidity, member **140** imposes a generally circular arc shape for the entirety of the band **20**. In some embodiments of the present invention, rigidity of the band **140** functions to prevent the exterior diameter of the band **20** from changing during adjustment of the internal diameter of the loop.

Generally, an increase or reduction of the length of the tension element **132** results in reversible radial displacement at the internal periphery of the band **20**. This in turn translates into a variation of internal diameter of the loop from a fully open diameter to a fully closed diameter.

In various embodiments of the present invention, the diameter of the opening **137** formed by the band **20** may be between about 25 mm and about 35 mm in a fully dilated position (e.g., see FIG. 5C). The diameter of the opening **137** formed by the band **20** may be between about 15 mm and about 20 mm in a fully constricted position (e.g., see FIG. 5D).

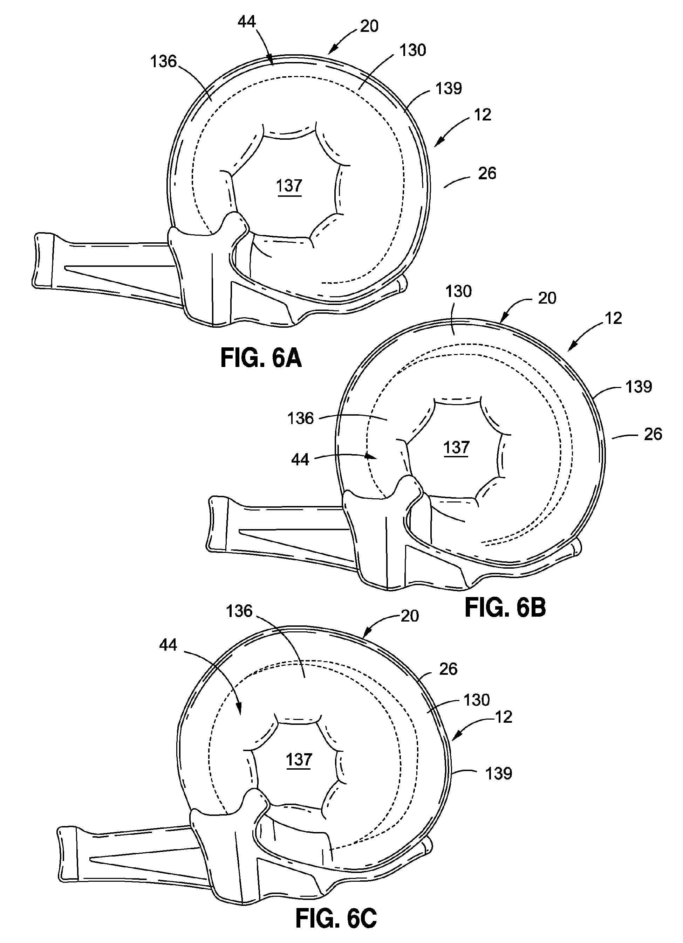

FIGS. 6A, 6B and 6C show the band **20** at progressively increased levels of constriction, with FIG. 6A showing the opening **137** being larger than in FIG. 6B, which shows the opening **137** larger than in FIG. 6C. In the shown embodiments, while the diameter of the opening **137** is adjustable, the diameter of an outer circumferential surface **139** of the band **20** remains relatively fixed during adjustments of the opening **137**. The membrane **45** of the contact region **44** stretches or unfolds as described elsewhere herein, as axially compressible material **136** moves apart from the distal element **130** and the band (not visible in FIGS. 6A-6C) and opening **137** constricts (see also FIG. 5D).

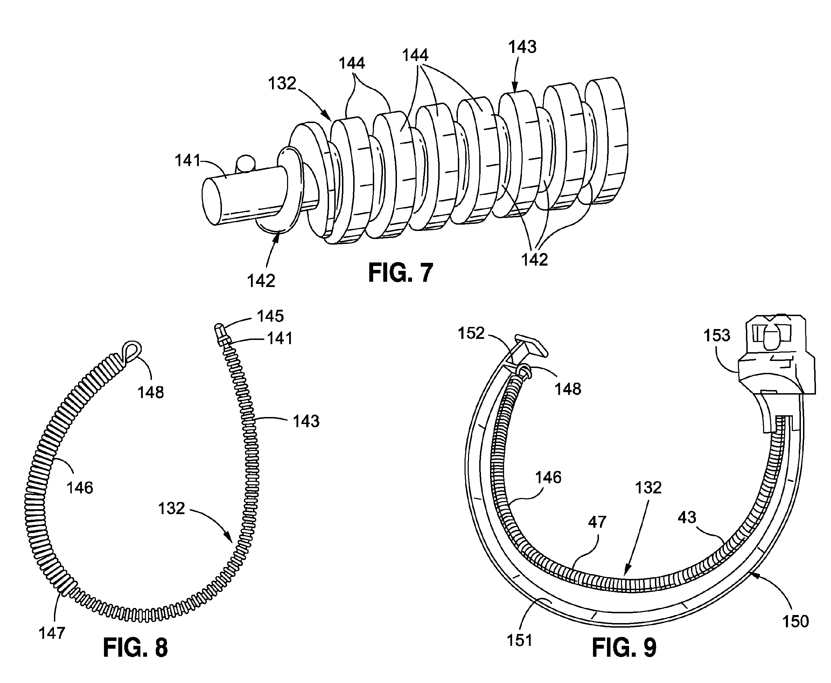

Referring now to FIG. 7, the tension element **132** is described. In some embodiments, the tension element **132** has sufficient flexibility to permit it to be formed into a substantially circular shape, while also being able to transmit the force necessary to adjust the inner diameter of the loop. The tension element **132** may comprise a flexible core **141,** for example, comprising a metal alloy wire of circular cross section, on which is fixed, and wound coaxially, at least one un-joined coil spring which defines a screw thread pitch.

The tension element **132** may comprise two unjoined coil springs that form a screw thread: first spring **142,** wound helicoidally along the flexible core **141,** and second spring **143** of greater exterior diameter. The second spring **143** preferably comprises coils **144** of rectangular transverse section, so as to delineate a flat external generatrix. The first spring **142** is interposed between the coils **144** of the second spring **143** to define and maintain a substantially constant square screw thread pitch, even when the tension element is subjected to bending.

The second spring **143** may be made by laser cutting a cylindrical hollow tube, e.g., made from stainless steel, or alternatively, by winding a wire with a rectangular, trapezoidal or other cross-section. When helically intertwined with the first spring **142,** the coils **144** of the second spring **143** are activated with an intrinsic elastic compression force from the adjacent coils of the first spring **142.** The first spring **142** is intertwined between the coils of the second spring **143.** The first spring **142** is fixedly joined to the flexible core **141** at one end. At the second end, a crimped cap **145** (see FIG. 8) is located a short distance from the ends of the springs **142** and **143** to allow for small extensions, for example, to accommodate flexion of the tension element **132** and/or to limit this extension to keep the thread pitch substantially constant.

Referring now to FIG. 8, a free end of the tension element **132** includes a crimped cap **145.** The second spring **143** includes coils having a square transverse section. The flexible core **141** extends through the first and second springs **142** and **143,** and terminates close to the crimped cap **145.** In one embodiment of the present invention, the tension element **132** further comprises a third spring **146** that is coupled to the flexible core **141,** and the first and second springs **142** and **143** at junction **147.** The third spring **146** includes a loop **148** at the end opposite to junction **147,** which permits the tension element **132** to be fixed at the first end **22** of the band **20** (see also FIG. 5A).

With respect to FIG. 9, the tension element **132** is shown disposed within a skeleton **150** of the band **20.** The skeleton **150** includes a layer **151** that forms a distal periphery, an anchor **152** that accepts the loop **148** of the tension element **132,** and an actuator housing **153.** The skeleton **150** may be made of a high strength moldable plastic.

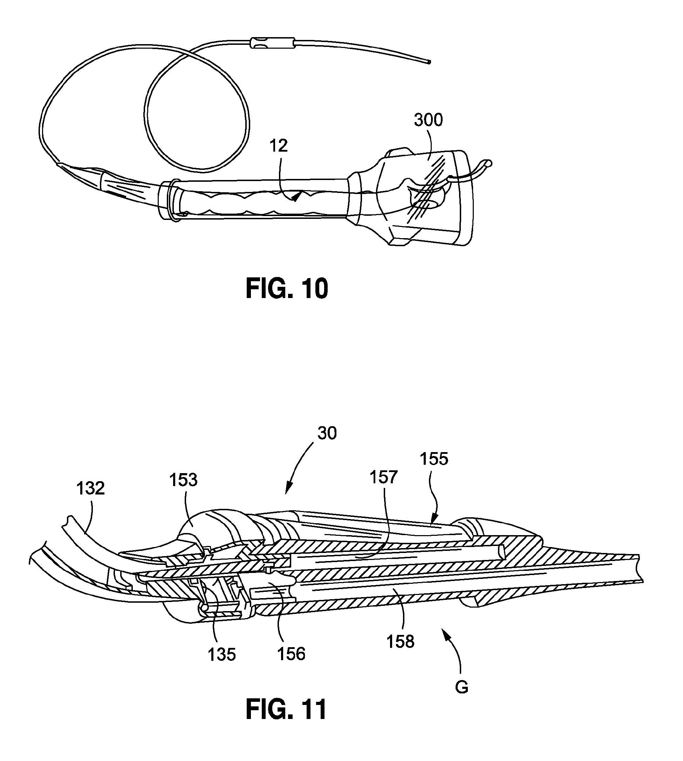

The third spring **146** permits the band **20** to be straightened for insertion through a trocar, for example, an 18 mm trocar, despite a differential elongation of the skeleton **150** and the tension element **132.** This feature is illustrated in FIG. 10 which shows the implantable portion **12** (e.g., the band **20**) disposed in a trocar **300** in order to facilitate laparoscopic implantation of the band **20.**

Referring now to FIG. 11, in the shown embodiment, a connector **30** includes a housing **155** having a recessed portion **156,** a tension element cavity **157** and a cable lumen **158.** The recessed portion **156** is configured to accept the actuator housing **153** of the skeleton **150,** so that as the tension element **132** is drawn through the actuator **135** it extends into a tension element cavity **157.** The cable lumen **158** extends through the housing **155** so that the cable may be coupled to the actuator **135.** The housing **155** may be grasped in area G using atraumatic laparoscopic graspers during implantation.

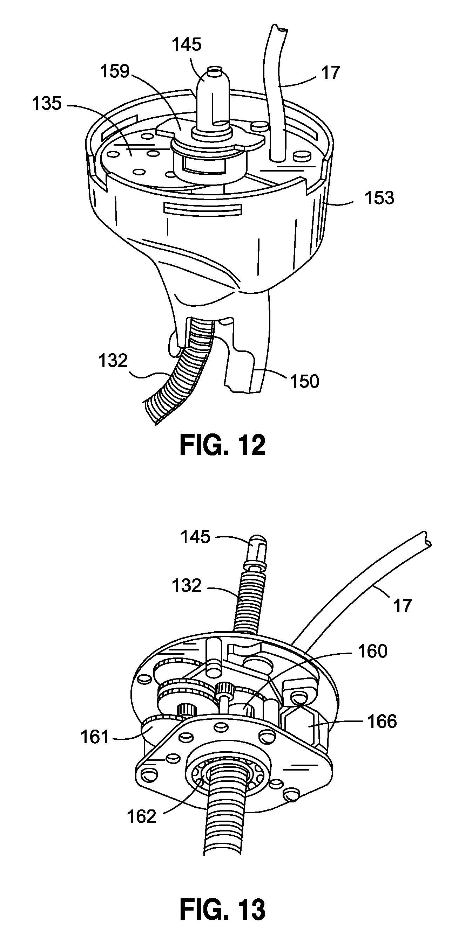

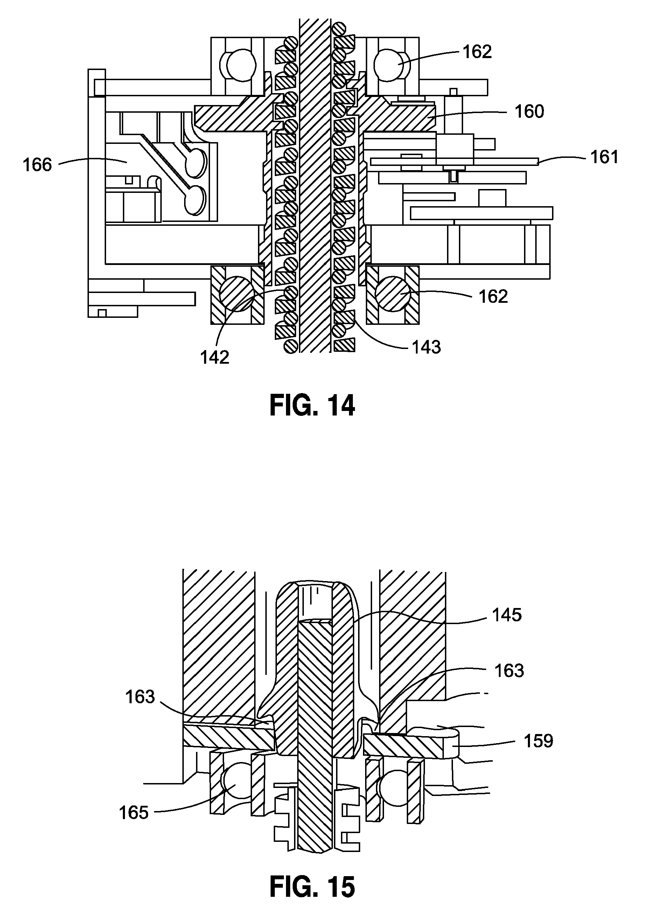

In FIG. 12, the actuator housing **153** of the skeleton **150** is shown with the actuator **135** and the tension element **132** disposed therethrough. The antenna cable **17** is coupled to a motor (not shown) disposed within the actuator housing **153.** The tension element **132** is in the fully opened (largest diameter) position, so that the crimped cap **145** contacts a printed circuit board **159** of the reference position switch described below with respect to FIG. 15.

With respect to FIGS. 13 and 14, the actuator **135** includes a motor **166** coupled to the antenna cable **17** that drives a nut **160** through gears **161.** The nut **160** is supported by upper and lower bearings **162** to minimize energy losses due to friction. The nut **160** is self-centering, self-guiding and provides high torque-to-axial force transfer. In addition, the nut **160** is self-blocking, meaning that the nut **160** will not rotate due to the application of pushing or pulling forces on the tension element **132.** This condition may be achieved by ensuring that the height (h) of the thread divided by the circumference of the screw ($2\pi R$) is less than the arctangent of the friction coefficient (p):

$$h/(2\pi R) < \arctan(\mu)$$

The gears **161** preferably are selected to provide good mechanical efficiency, for example, with a reduction factor greater than 1000. In addition, the volume of the actuator depicted in FIGS. 13 and 14 may be quite small, with a total volume less than 1 $cm^3$ and a diameter less than 12.5 mm, so that the device may easily pass through a standard trocar. In a preferred embodiment, the gears **161** are selected to provide a force of more than 2 kg on the screw thread of the tension element **132** at an electrical consumption of only 50 mW. The gears **161** and other components of the actuator **135** may be made of stainless steel or other alloys like Arcap (CuNiZn), or can be gold plated to permit operation in the high humidity likely to be encountered in a human body.

The motor **166** employed in the actuator **135** may comprise a Lavet-type high precision stepper motor with a flat magnetic circuit, such as are used in watches. The motor **166** may be a two phase (two coil) motor that permits bi-directional rotation, has good efficiency, and may be supplied with a square wave signal directly by the microcontroller circuitry within antenna/controller pod **15,** thus eliminating the need for an interface circuit. Alternatively, the motor employed in the actuator **135** may be of a brushless DC type motor. In addition, the motor preferably is compatible with magnetic resonance imaging, i.e., remains functional when exposed to strong magnetic fields used in medical imaging equipment.

Referring now to FIG. 15, a reference position switch of an embodiment of the present invention is described. In one embodiment the actuator of the present invention employs the nut **160** driven by a stepper motor. Thus, there is no need for the system to include a position sensor or encoder to determine the length of the tension element **132** drawn through the actuator **135.** Instead, the diameter of the opening **137** may be computed as a function of the screw thread pitch and the number of rotations of the nut **160.** At least one reference datum point may be provided which may be calculated by using a reference position switch that is activated when the band **20** is moved to its fully open position. The crimped cap **145** on the free end of the tension element **132** may be used to serve this function by contacting the electrical traces **163** on the printed circuit board **159** (and also limits elongation of the screw thread). The circuit board **159** is disposed just above the bearing **165,** which forms part of the actuator **135.** When the crimped cap **145** contacts the electrical traces **163** it closes a switch that signals the implantable controller that the band **20** is in the fully open position.

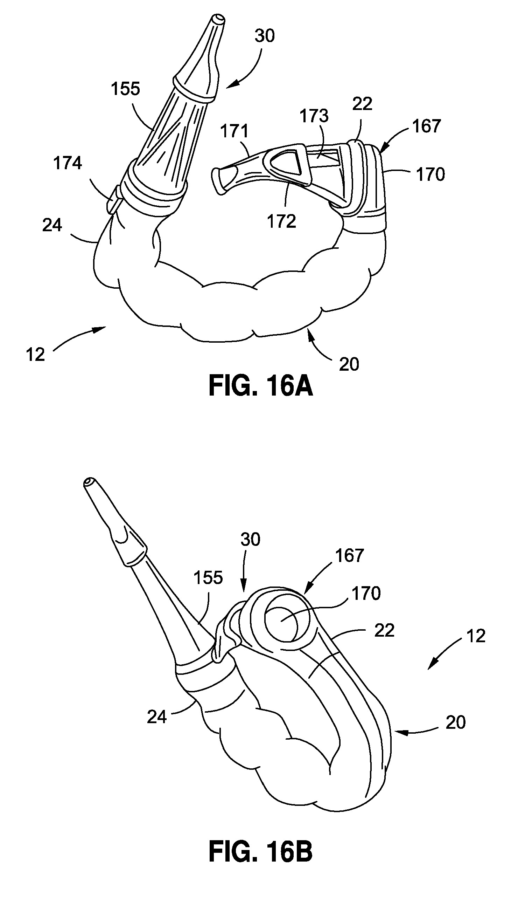

Referring now to FIGS. 16A and 16B, a clip **30** may include a clip element **167** on the first end **22** of the band **20** and the housing **155** on the second end of the band **20.** The clip element **167** includes an aperture **170,** a tab **171** having a hinge **172** and a slot **173.** The aperture **170** is dimensioned to accept the housing **155** on the second end **24** of the band **20,** while the slot **173** is dimensioned to accept a flange **174** disposed on the second end **24.**

An example of a method of coupling the first end **22** with the second end **24** during implantation of the band **20** is now described. To couple the first end **22** and the second end **24,** the clip element **167** is grasped by the tab **171,** and the tag **18** of the pod **15** (see FIG. 1) is inserted through the aperture **170.** The clip element **167** is then pulled towards the second end **24** so that the housing **155** passes through the aperture **170** while the housing **155** is grasped with atraumatic forceps; the conical shape of the housing **155** facilitates this action. The force is applied to the tab **171** until the slot **173** captures the flange **174,** thereby securing the first and second ends **22, 24** in the closed position. The physician may subsequently choose to disengage the slot **173** from the flange **174** by manipulating the tab **171** using laparoscopic forceps, for example, to reposition the band **20.** In some embodiments, forces inadvertently applied to the tab **171** in an opposite direction cause the tab **171** to buckle at the hinge **172,** but do not cause the flange **174** to exit the slot **173.** Accordingly, the hinge **172** of the tab **171** prevents accidental opening of the clip **30** when the tab **171** is subjected to forces that cause the tab **171** to fold backwards away from the housing **155** such as may arise due to movement of the patient, the organ, or bolus of fluid passing through the organ.

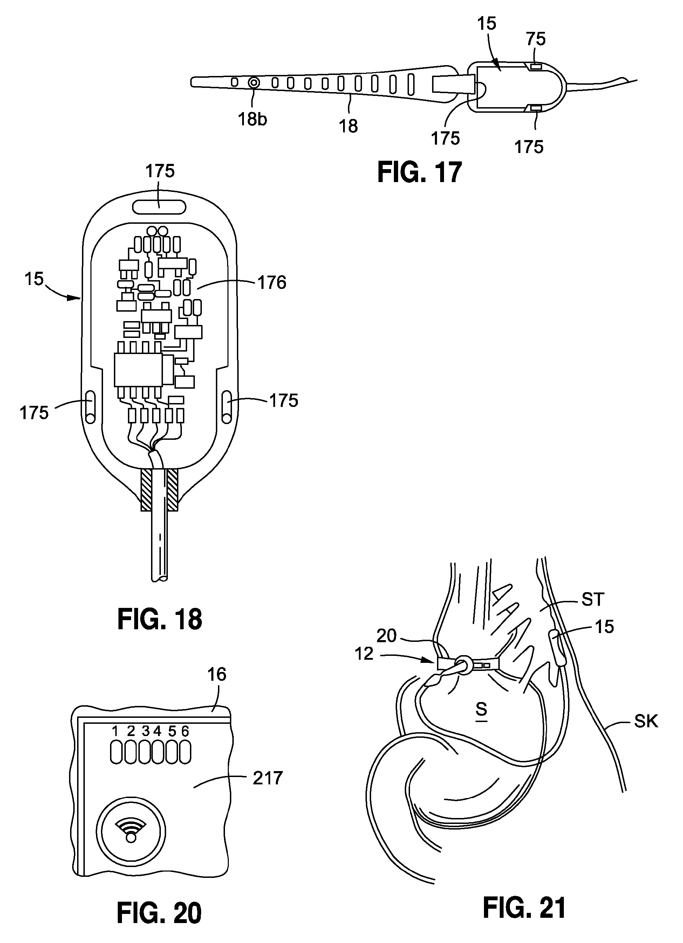

With respect to FIGS. 17 and 18, the removable tag **18** of the antenna/controller pod **15** may include apertures **175.** The tag **18** comprises a grip structure that facilitates manipulation and placement of the pod **15** during implantation; after which the tag **18** is removed, for example, using a scissors cut. The tag **18** also includes aperture **18b** that allows the use of a suture thread to assist in passing the antenna/controller pod **15** behind the stomach. The holes **175** also are dimensioned to be compatible with standard suture needles from size 1-0 to 7-0 to permit the pod **15** to be sutured to the patient's sternum, thereby ensuring that the pod **15** remains accessible to the external antenna and cannot migrate from a desired implantation site.

As shown in FIG. 18, the antenna/controller pod **15** encloses the printed circuit board **176** that carries the antenna and microcontroller circuitry of the band (not shown). The antenna receives energy and commands from the external control **16** (see FIG. 1), and supplies those signals to the microcontroller, which in turn powers the motor **166** of the actuator **135** (FIGS. 12 and 13). The circuitry of the antenna/controller pod 15 uses the energy received from the incoming signal to power the circuit, interprets the commands received from the external control 16, and supplies appropriate signals to the motor **166** of the actuator **135.** The circuit also retrieves information regarding operation of the motor **166** of the actuator **135** and relays that information to the external control **16** via the antenna. The printed circuit board **176** may be covered with a water-resistant polymeric covering, e.g., Parylene, to permit use in the high (up to 100%) humidity environment encountered in the body.

The antenna/controller pod **15** may include a mechanical closure system that is augmented by silicone glue so that the pod **15** is fluid tight. This silicone glue also is used to protect the soldered wires.

The actuator **135** may be linked to subcutaneous antenna/controller pod **15** to receive a radio frequency control and power signal. In one embodiment, the motor **166** of the actuator **135** has no internal energy supply, but rather is powered by the receiving circuit of the antenna through a rechargeable energy storage device, such as a capacitor. For example, the receiving circuit converts radio frequency waves received from external control **16** via the antenna into a motor control and power signal. In another embodiment the actuator **135** may be driven via an implantable rechargeable battery.

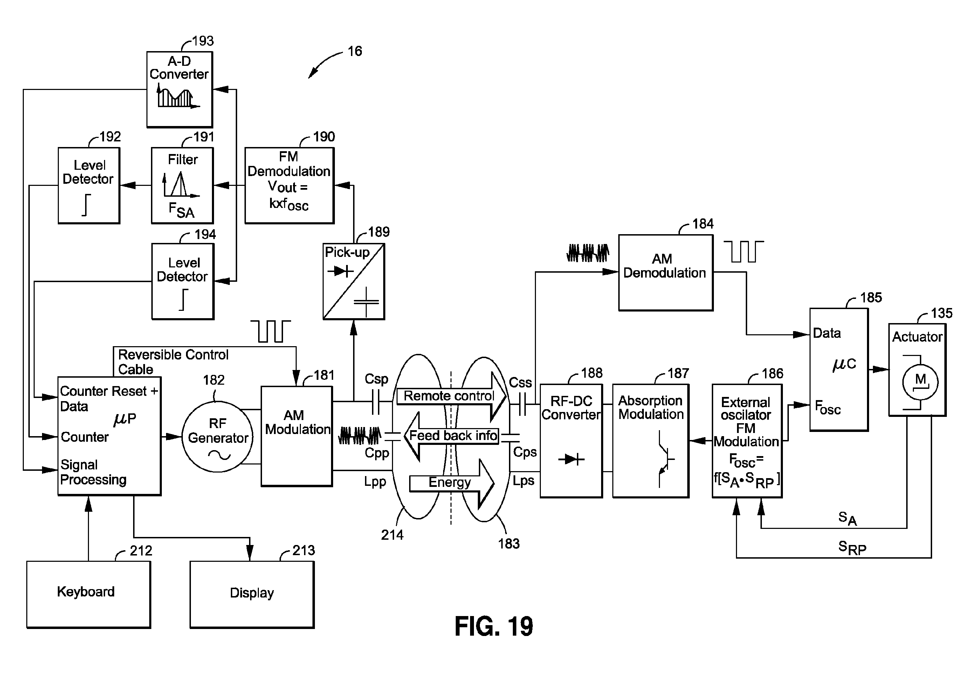

Referring to FIG. 19, one suitable arrangement of circuitry that may be employed in the external control **16** of the present invention is described herein. The external control **16** includes a microprocessor **180** coupled to a keyboard/control panel **212** and a display **213.** The external control **16** produces a signal comprising one or more data bytes to be transmitted to the implantable antenna/controller pod (not shown) and the actuator **135.**

The external control **16** includes a modulator **181** for amplitude modulation of the RF wave from a RF generator **182,** whose signal is emitted by an external antenna **214.** The emitted signal or wave is received by the antenna **183** in the antenna/controller pod (not shown), where the AM demodulator **184** extracts the data bytes from the envelope of the received RF signal. The data bytes then are decoded by the microcontroller **185.** A special code is used that allows easy decoding of the data by the microcontroller **185,** but also provides maximal security against communication failure.

The external oscillator **186,** which is a voltage controlled oscillator (VCO), provides a clock signal to the microcontroller **185**. The oscillator **186** may comprise, for example, a relaxation oscillator comprising an external resistor-capacitor network connected to a discharging logic circuitry already implemented in the microcontroller or a crystal oscillator comprising a resonant circuit with a crystal, capacitors and logic circuits.

The microcontroller **185** interprets the received instructions and produces an output that drives the motor of the actuator **135**. As discussed above, the actuator **135** may comprise a bi-directional stepper motor that drives the nut **160** through a series of reducing gears. In one embodiment, the two coils of the stepper motor of the actuator **135** are directly connected to the microcontroller **185,** which receives the working instructions from the demodulator **184,** interprets them and provides the voltage sequences to the motor coils. When the supply of voltage pulses to the stepper motor stops, the gears are designed to remain stationary, even if a reverse torque or force is applied to the nut **160** by the tension element **132.**

As also described above, use of a stepper motor in the actuator **135** makes it is possible to obtain positional information on the nut **160** and the tension element **132** without the use of sensors or encoders, because the displacement of the tension element **132** is proportional to the number of pulses supplied to the stepper motor coils. Two signals may be employed to ensure precise control, reference position signal $S_{RP}$, generated by the reference position switch of FIG. 15, and the actuator signal $S_A$.

According to one embodiment, signal $S_A$ is the voltage signal taken at one of the outputs of the microcontroller **185** that is connected to the motor coils of the actuator **135.** Alternatively, signal $S_A$ can be derived from the current applied to a motor coil instead of the voltage, or may be an induced voltage on a secondary coil wrapped around one of the motor coils of the actuator **135.** In either case, signal $S_A$ may be a pulsating signal that contains information on the number of steps turned by the rotor and further indicates whether blockage of the mechanism has occurred. Specifically, if the rotor of the stepper motor fails to turn, the magnetic circuit is disturbed, and by induction, affects signal $S_A$, e.g., by altering the shape of the signal. This disturbance can be detected in the external control, as described below.

The signals $S_A$ and $S_{RP}$ are converted into frequencies using the external oscillator **186,** so that the voltage level of signal $S_A$ applied to the external oscillator **186** causes the oscillator to vary its frequency $F_{OSC}$ proportionally to the signal $S_A$. Thus, $F_{OSC}$ contains all the information of signal $S_A$. When the crimped cap **145** and the tension element **132** are in the reference position (band **20** is fully open), the reference position switch produces reference position signal $S_{RP}$. The signal $S_{RP}$ is used to induce a constant shift of the frequency $F_{OSC}$, which shift is easily distinguishable from the variations due to the signal $S_A$.

If the oscillator **186** is a relaxation oscillator, as described above, the signals $S_A$ and $S_{RP}$ modify the charging current of the external resistor capacitor network. In this case, the relaxation oscillator may comprise an external resistor-capacitor network connected to a transistor and a logic circuit implemented in the microcontroller **185**. With the signals $S_A$ and $S_{RP}$, the goal is to modify the charging current of the capacitor of the RC network to change the frequency of the relaxation oscillator. If the charging current is low, the voltage of the capacitor increases slowly and when the threshold of the transistor is reached, the capacitor discharges through the transistor. The frequency of the charging-discharging sequence depends on the charging current.

If the oscillator **186** is a crystal oscillator, signals $S_A$ and $S_{RP}$ modify the capacitor of the resonant circuit. In this case, the crystal oscillator circuit preferably comprises a crystal in parallel with the capacitors, so that the crystal and the capacitors form a resonant circuit which oscillates at a fixed frequency. This frequency can be adjusted by changing the capacitors. If one of these capacitors is a Varicap (a type of diode), it is possible to vary its capacitance value by modifying the reverse voltage applied on it, signals $S_A$ and $S_{RP}$ can be used to modify this voltage. In either of the foregoing cases, signals $S_A$ and $S_{RP}$ may be used to modify at least one parameter of a resistor-capacitor (RC) network associated with the oscillator **186** or at least one parameter of a crystal oscillator comprising the oscillator **186.**

Referring still to FIG. 19, signals $S_A$ and $S_{RP}$, derived from the stepper motor or from the output of the microcontroller **185,** may be used directly for frequency modulation by the oscillator **186** without any encoding or intervention by the microcontroller **185.** By using the oscillator **186** of the microcontroller **185** as part of the VCO for the feedback signal, no additional components are required, and operation of the microcontroller **185** is not adversely affected by the changes in the oscillator frequency $F_{OSC}$. The oscillating signal $F_{OSC}$ drives the voltage driven switch **187** for absorption modulation, such that feedback transmission is performed with passive telemetry by FM-AM absorption modulation.

More specifically, the signal $F_{OSC}$ drives switch **187** such that during the ON state of the switch **187** there is an increase in energy absorption by the RF-DC converter **188**. Accordingly, therefore the absorption rate is modulated at the frequency $F_{OSC}$ and thus the frequency of the amplitude modulation of the reflected signal or wave detected by the external control **16** contains the information for signal $S_A$. As discussed below, the pickup **189** in the external control **16** separates the reflected signal or wave where it can be decoded by FM demodulation in the demodulator **190** to obtain signal $S_{A'}$. This method therefore allows the transmission of different signals carried at different frequencies, and has the advantage that the ON state of the switch **187** can be very short and the absorption very strong without inducing an increase in average consumption. In this way, feedback transmission is less sensitive to variation in the quality of coupling between the antennas **183** and **214.**

In the external control **16,** the feedback signal $F_{OSC}$ is detected by the pickup **189** and fed to the FM demodulator **190,** which produces a voltage output $V_{OUT}$ that is proportional to $F_{OSC}$. $V_{OUT}$ is fed to the filter **191** and the level detector **192** to obtain the information corresponding to the actuator signal $S_A$, which in turn corresponds to the pulses applied to the stepper motor coil. The microprocessor **180** counts these pulses to calculate the corresponding displacement of the tension element **32,** which is proportional to the number of pulses.

The signal $V_{OUT}$ also is passed through the analog-to-digital converter **193** and the digital output is fed to the microprocessor **180,** where signal processing is performed to detect perturbations of the shape of the feedback signal that would indicate a blockage of the rotor of the stepper motor. The microprocessor **180** stops counting any detected motor pulses when it detects that the actuator is blocked, and outputs an indication of this status. The level detector **194** produces an output when it detects that the demodulated signal $V_{OUT}$ indicates the presence of the reference position signal $S_{RP}$ due to activation of the reference position switch. This output induces a reset of the position of the tension element calculated by the microprocessor **180** in the external control. In this way, a small imprecision, e.g. an offset, can be corrected.

As described above, the external control **16** may be configured to transmit both energy and commands to the implantable controller circuitry in the antenna/controller pod **15.** The external control **16** may also receive feedback information from the implantable controller that can be correlated to the position of the tension element **132** and the diameter of the loop. The external control **16** and the implantable controller may be configured in a master-slave arrangement, in which the implantable controller is completely passive, awaiting both instructions and power from the external control **16.**

Power may be delivered to the implantable pod **15** via magnetic induction. The quality of the coupling may be evaluated by analyzing the level of the feedback signal received by the external control **16,** and a metric corresponding to this parameter may be displayed on the signal strength indicator **217** on the control **16,** which in the shown embodiment, includes 6 LEDs (corresponding to six levels of coupling). If the coupling between the antennae is insufficient, the motor of the actuator may not work properly.

Referring now to FIG. 21, the band **20** of the presently described system is shown implanted in a patient. The band **20** is disposed encircling the upper portion of the patient's stomach S while the antenna/controller pod **15** is disposed adjacent to the patient's sternum ST. The pod **15** is located in this position beneath the patient's skin SK so that it is easily accessible in the patient's chest area to facilitate coupling of the implanted pod **15** to an external antenna of the control **16.**

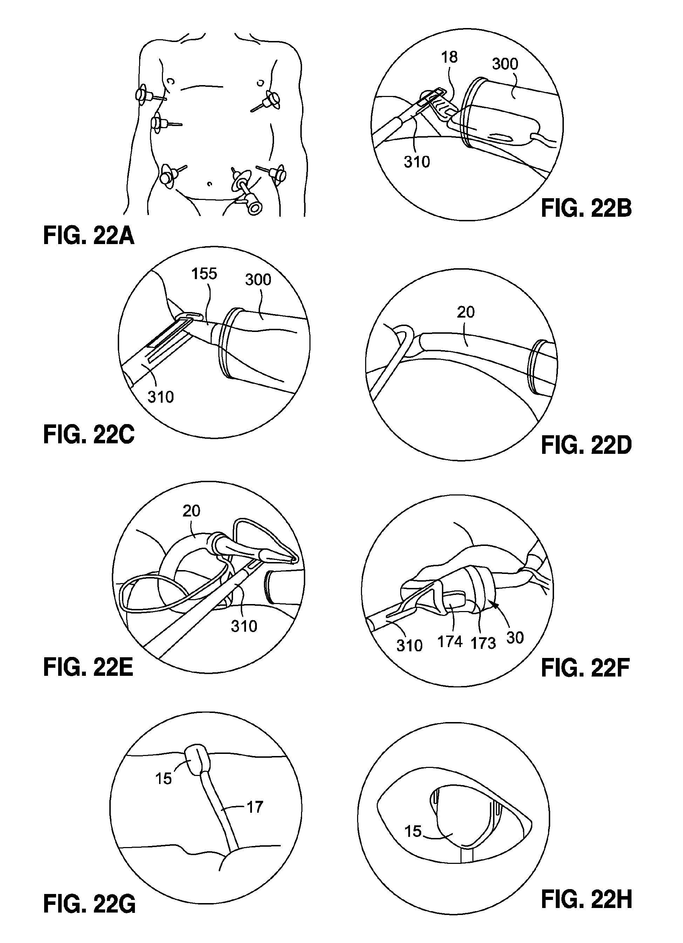

Referring to FIGS. 22A to 22H, a method of implanting the band and the pod of the system of the present invention is described. The method is similar to laparoscopic procedures used to implant previously-known hydraulically-actuated gastric bands.

Access to the abdomen is obtained by using 4 to 6 small holes, generally 10 to 18 mm in diameter, with a trocar inserted in each hole, as depicted in FIG. 22A. A camera and laparoscopic surgical tools are introduced and manipulated through the trocars. In addition, to permit free motion of the surgical tools and camera, the abdomen is inflated with $CO_2$ to an overpressure of approximately 0.15 bars.

In FIGS. 22B-22E, the band **20** of the implantable portion **12** is straightened (as depicted in FIG. 10) and inserted, antenna first, into the abdomen through an 18 mm trocar. Alternatively, a laparoscopic cannula may be used to make an incision and then withdrawn, and the device is inserted through the opening so created (other instruments also may be used to form this laparotomy). In FIG. 22B, the tag **18** of the antenna/controller pod **15** is shown entering the abdomen through the trocar **300** using atraumatic graspers **310.** In FIG. 22C, the housing **155** is shown being drawn into the abdomen through trocar **300,** again using atraumatic graspers **310.** FIG. 22D shows the band **20** entering the abdomen in an extended position. In FIG. 22E, the band **20** is permitted to resume its arcuate shape.

The band **20** then is manipulated using atraumatic graspers **310** as described elsewhere herein, to secure the band **20** around the upper portion of the patient's stomach until the slot **173** of the clip **30** is engaged with the flange **174,** as shown in FIG. 22F. A fold of stomach tissue then may be sutured around the band **20** to prevent migration of the band **20.**

Finally, as shown in FIG. 22G, a channel may be formed through the abdominal wall and the antenna/controller pod **15** passed through the channel. The tag **18** then is cut off of the antenna/controller pod **15,** and the pod **15** is sutured into position above the patient's sternum, as depicted in FIG. 22H. The trocars then are removed, and the band **20** may be activated to adjust the diameter of the inner diameter as desired by the physician.

The process of removing the band **20** involves substantially reversing the sequence of steps described above, and may be accomplished non-destructively. In particular, a plurality of cannulae into the abdominal cavity and the abdominal cavity then insufflated to create a pneumoperitoneum. Using laparoscopic graspers, the clip **30** may be unclipped and the band **20** removed from a position encircling the patient's stomach. The band **20** may then be straightened and withdrawn from the abdominal cavity either through one of the plurality of cannulae or via a laparotomy.

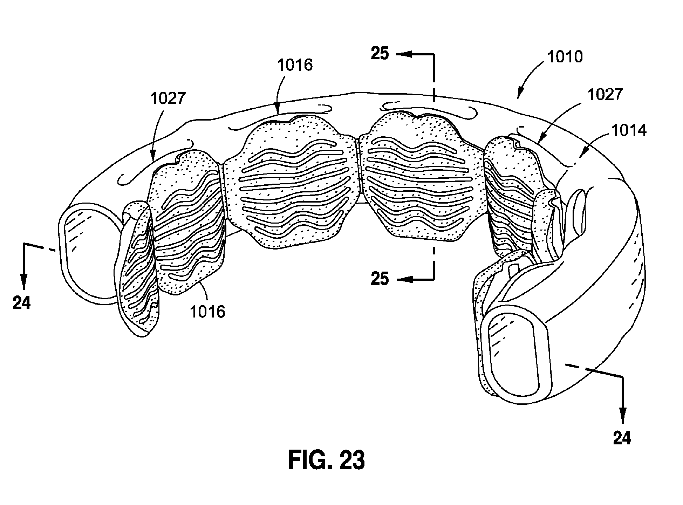

FIGS. 23 through 25 illustrate an alternative contact region **1010** of a gastric banding system of the present invention. The contact region **1010** may be identical to the contact region **44** except as explicitly described below. The contact region **1010** can replace the contact region **44** described and shown, for example, in FIGS. 3 and 3A, in the system **10.**

The contact region **1010** comprises a membrane **1014** which may be substantially identical to the membrane **45** described and shown elsewhere herein. In this embodiment however, cushions **1016,** which may be made of the same incompressible materials as cushions **60,** are affixed to an external surface of the membrane **1014** and define at least a portion of the stomach-facing surface of the contact region **1010.** The cushions **1016** may be individually molded to, or molded as a whole, directly to the membrane **1014** using conventional molding techniques, for example, conventional overmolding techniques.

In a specific embodiment, the cushions **1016** are made of silicone elastomer having a hardness of about 10 Shore A and the membrane **1014** is made of silicone elastomer having a hardness of about 30 Shore A.

Alternatively, the membrane **1014** may be made of a silicone elastomer of a different hardness, such as, for example, about 20 Shore A to about 45 Shore A. Alternatively still, the cushions can be made of an even softer silicone elastomer, such as about 5 Shore A or about 1 Shore A. Alternatively, the cushions or the membrane can be made of other suitable implantable materials.

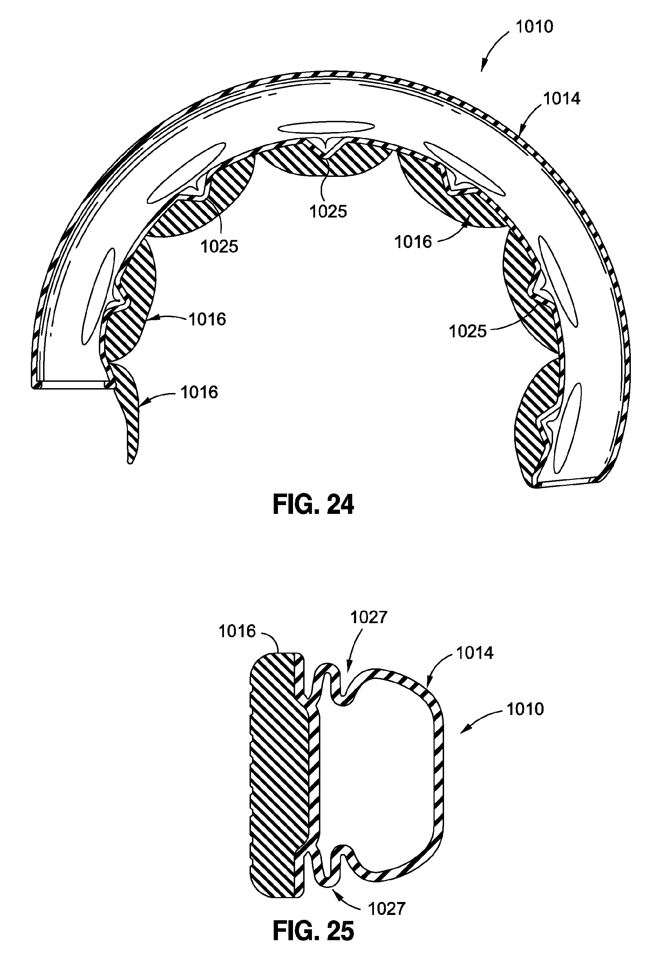

FIGS. 24 and 25 are cross-sectional views of the contact region shown in FIG. 23 taken along line 24-24 and line 25-25, respectively.

Another feature of this embodiment of the invention is shown in FIG. 24. Specifically, the membrane **1014** may includes a structural support, for example, a wedge **1025** located at the interface between the membrane **1014** and each of the cushions 1016. The wedges **1025** may provide an increased surface area on which the cushions are molded thereby providing additional adherence and/or support between the membrane **1014** and the cushions **1016.** Like membrane **45,** the membrane **1014** includes corrugations **1027** for facilitating unfolding or expansion of the membrane **1014** during adjustment of the band **20.**

Another advantageous feature of this embodiment is shown in FIGS. 26-27A. In some embodiments, the cushions **60** and the tension segments **52** form an inner circumference of the loop configuration having a generally star-shape, defined by the contact region, as shown in FIG. 26. The stomach lumen is indicated by numeral **1033.** During constriction of the band, which is shown dilated in FIGS. 26 and 26A and constricted in FIGS. 27 and 27A, the adjacent incompressible cushions **60** form a progressively narrowing angle, for example, a progressively narrowing substantially V-shaped surface having convex, arcuate surfaces defined by the cushions **60.** The tension segments **52** are located between the adjacent cushions **60** and form the vertices of the angles.

While not wishing to be bound by any particular theory of operation, it is believed that the structure of the contact member **44** and at least partially due to the incompressibility of the cushions **60** enables the band **20** to constrict about the stomach without pinching the tissue. For example, as shown in FIGS. 27 and 27A, the stomach tissue does not become entrapped between adjacent cushions **60.** During constriction of the band **20,** the convex stomach-facing surfaces maintain their shape and form no gaps, while folding inwardly toward one another. This mechanism and structure causes the tissues of the stomach constricted without the tissues becoming entrapped and/or pinched. This progressive V-shape acts differently from mechanical pliers.

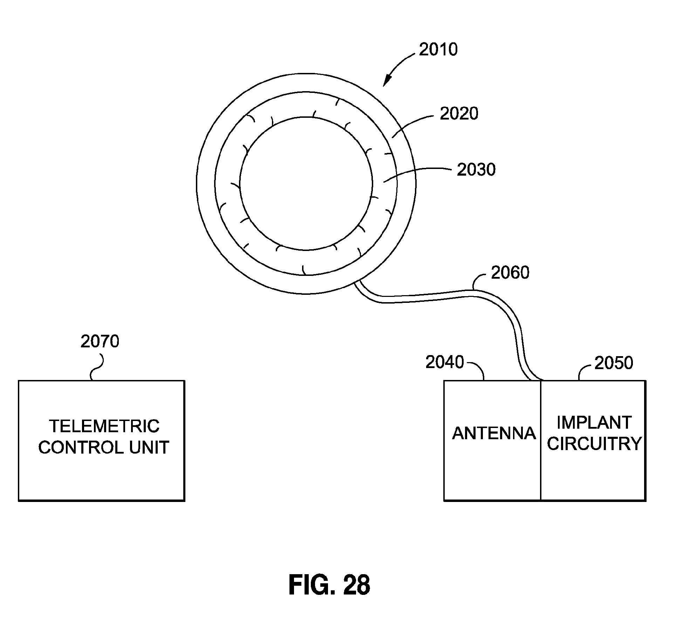

FIGS. 28 through 32 illustrate yet another alternative contact region of a gastric banding system of the present invention, such as an inflatable compartment **2030.** The inflatable compartment **2030** may be identical to the contact region **44** except as explicitly described below. The inflatable compartment **2030** can replace the contact region **44** described and shown, for example, in FIGS. 3 and 3A, in the system **10.** In exemplary embodiments, the inflatable compartment **2030** may be used in connection with the exemplary inner mechanism shown in FIGS. 5A-5D which enables adjustment of the inner circumference of the loop configuration.

Preliminarily, and as already discussed herein, obesity is a matter of worldwide concern. Various approaches have been taken to address the underlying causes of obesity, including adjustable gastric banding. There are at least three types of adjustable gastric bands, namely, remotely adjustable bands (RABs), hydraulic adjustable bands (HABs), and hydraulic remotely adjustable bands (HRABs), each having its own respective advantages and disadvantages.

An exemplary RAB, such as described herein, comprises a stepper motor which rotates around a flexible screw within the ring of the band to adjust the band through a variety of diameters. As the stepper motor drives forward it reduces the stoma of the band and when it reverses it increases the stoma of the band. The adjustment is telemetrically controlled by an external controller. The band has an EPTFE cushion which is covered by a silicone sheath to contact the stomach. While RABs are relatively low profile, the stomach interface is not as soft as with HABs and HRABs, there is a greater potential for slippage than with HABs and HRABs, and there is no override mechanism in the event of an emergency as with HABs and HRABs.

An exemplary HAB comprises saline solution inside of one or more inflatable silicone shells positioned on the stomach surface of the ring of the band to adjust the band through a variety of diameters. As the shell is inflated it reduces the stoma of the band and when it is deflated it increases the stoma of the band. Saline solution is added or removed from the shell via an access port fixed beneath the skin of the patient in the abdomen on the rectus muscle sheath using a fine needle to find the right level of restriction. While HABs are characterized by a soft saline solution stomach interface, with which physicians are very comfortable, and may incorporate an override mechanism in the event of an emergency, HABs are bulkier than RABs and the access port bump is often unattractive, especially after patients lose weight.

An exemplary HRAB also comprises saline solution inside of one or more inflatable silicone shells positioned on the stomach surface of the ring of the band to adjust the band through a variety of diameters. However, rather than via an access port fixed beneath the skin of the patient's abdomen, saline solution is added or removed from the shell via a pump, active valves, electronics, and reservoir. Similar to HABs, while HRABs are characterized by a soft stomach interface and may incorporate an override mechanism in the event of an emergency, the components of HRABs require a larger overall implant size than both HABs and RABs, which is viewed as a disadvantage.

An object of the present invention is therefore to combine the best features of RABs, HABs, and HRABs in a novel and non-obvious manner to thereby reduce the disadvantages of each design.

With reference to FIG. 28, an implantable banding system in accordance with exemplary embodiments of the present invention comprises a gastric band **2010** and: (i) a mechanical adjustment mechanism **2020,** (ii) one or more inflatable compartments **2030,** (iii) an antenna **2040** and implant circuitry **2050,** (iv) a connector **2060,** and (v) a telemetric control unit **2070.** The inflatable compartments **2030** can include, for example, a fixed volume of fluid. Not shown in FIG. 28, an implantable banding system in accordance with exemplary embodiments of the present invention may optionally further comprise, inter alia, one or more of: (i) an override mechanism, (ii) a pressure monitoring mechanism, (iii) a self-adjusting mechanism, and (iv) a drug delivery mechanism.

An implantable banding system in accordance with exemplary embodiments of the present invention comprises a mechanical adjustment mechanism **2020,** the same as or similar to the exemplary inner mechanism shown in FIGS. 5A-5D which enables adjustment of the inner circumference of the loop configuration. Exemplary mechanical adjustment mechanisms are configured to adjust the gastric band **2010** through a variety of diameters. In accordance with exemplary embodiments, the mechanical adjustment mechanism **2020** comprises an outer portion of the ring of the gastric band **2010** or implantable banding system. An exemplary mechanical adjustment mechanism **2020** comprises a stepper motor which rotates around a flexible screw within the ring of the band to adjust the gastric band **2010** through a variety of diameters. As the stepper motor drives forward it reduces the stoma of the band and when it reverses it increases the stoma of the band. The adjustment is telemetrically controlled by a telemetric control unit. An exemplary mechanical adjustment mechanism **2020** is described in U.S. Publication No. 2005/0143766, to Bachmann et al. In general, a mechanical adjustment mechanism **2020** is any device designed or otherwise configured to controllably restrict the band circumferentially about the stomach.

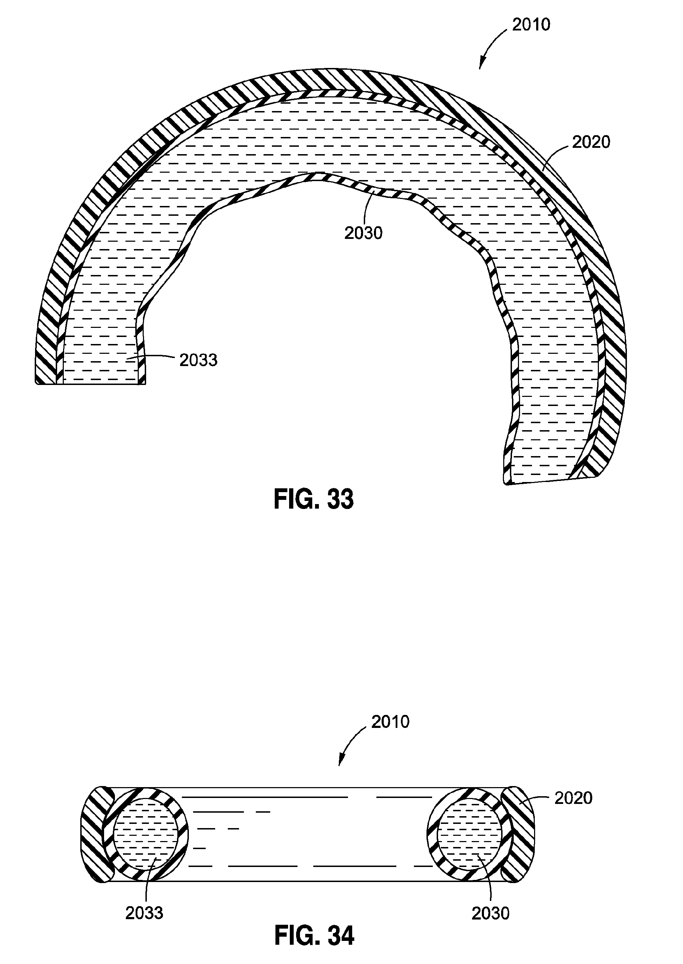

An implantable banding system in accordance with exemplary embodiments of the present invention comprises one or more inflatable compartments **2030.** Exemplary inflatable compartments **2030** are positioned on the stomach surface of the ring of the band and configured to provide a soft stomach interface. An exemplary inflatable compartment **2030** may comprise a silicone shell or balloon filled completely or partially with a fluid, e.g., saline solution, water or the like. The fluid can be, for example, a fixed volume of saline or a gel. The saline can be noncompressible, but soft. In accordance with exemplary embodiments, the fluid compartment comprises an inner portion of the ring of the gastric band **2010** or implantable banding system. In accordance with exemplary embodiments, the inner fluid compartment of the ring of the gastric banding element is bonded to, or overmolded onto, the outer mechanical adjustment mechanism either directly or with a silicone sheath intermediary element. As can be seen in the sectional views of the gastric band **2010** in FIGS. 33 and 34, the one or more inflatable compartments **2030** can be filled with fluid **2033.**

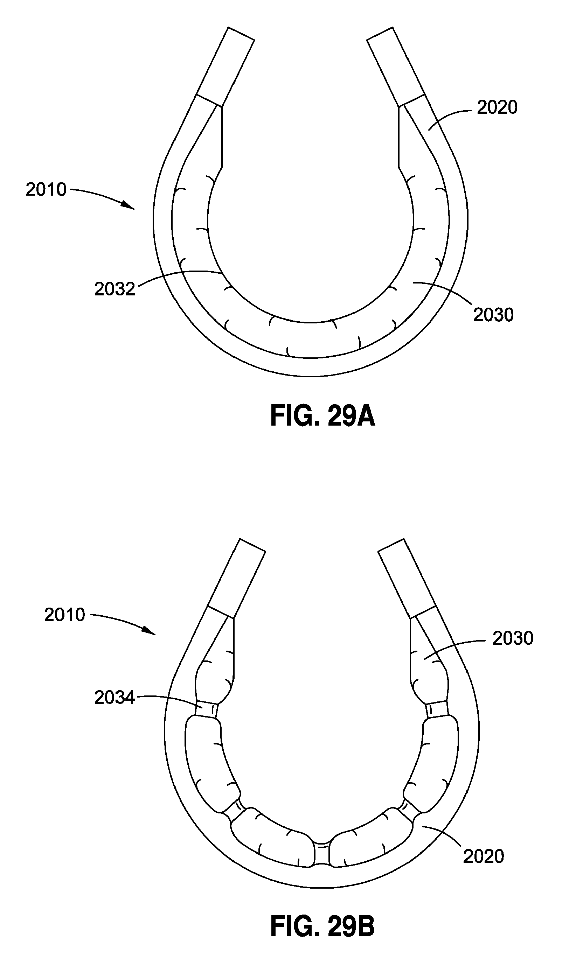

With momentary reference to FIGS. 29A and 29B, in various embodiments, an inflatable compartment **2030** may comprise one or more smooth features **2032** or fatigue-resistant features **2034,** such as those described in U.S. Publication No. 2005/0082793, to the present inventor Birk, and U.S. Publication No. 2005/0192531, to the present inventor Birk. In general, an infaltable compartment **2030** is any device designed or otherwise configured to be a cushioning element between the mechanical adjustment mechanism and the stomach.

An implantable banding system in accordance with exemplary embodiments of the present invention comprises an antenna **2040** configured to telemetrically communicate with the telemetric control unit, and implant circuitry **2050** configured to process information received by the antenna **2040** and thereby control the mechanical adjustment mechanism. In accordance with exemplary embodiments, the antenna **2040** is a low profile antenna or the like, and is attached to the tissue on top of the sternum. Even in morbidly obese patients, the tissue on top of the sternum is a consistent thickness and does not usually exceed several centimeters. By placing the antenna **2040** in this location, the distance between the implant and the telemetric control unit's antenna **2040** is short and a predictable distance. The shallow depth substantially reduces the power requirements necessary to power the band and allows for a smaller external inductive antenna **2040** to power the implant.

An implantable banding system in accordance with exemplary embodiments of the present invention comprises a connector **2060** through which the antenna **2040** and the implant circuitry **2050** are in communication with the mechanical adjustment mechanism **2020**.

In exemplary embodiments, the implantable banding system is telemetrically powered, e.g., by RF. In other embodiments, the implantable banding system is powered by one or more of a battery, rechargeable or otherwise, a capacitor, and a fuel cell. In accordance with various aspects of an exemplary embodiment, the power source is recharged by one or more of motion, a chemical change, and a temperature change. For example, in exemplary embodiments, the implantable banding system is powered by one or more of the following: (i) kinetic energy created by body motion stored onto a capacitor, (ii) an implanted fuel cell, (iii) an implanted power source powered by chemistry of the body, (iv) an implanted power source powered by temperature change, and (v) implanted batteries that can be recharged by direct contact.

An implantable banding system in accordance with exemplary embodiments of the present invention comprises a telemetric control unit **2070**. In exemplary embodiments, the telemetric control unit **2070** is configured to communicate remotely with the implant circuitry **2050** via the antenna **2040**. In exemplary embodiments, the telemetric control unit **2070** provides for wireless control of one or more of mechanical adjustment mechanism **2020,** the override mechanism, the pressure monitoring mechanism, the self-adjusting mechanism, and the drug delivery mechanism.

In exemplary embodiments, the telemetric control unit **2070** is powered by alternating current, direct current, e.g., one or more of a battery, rechargeable or otherwise, a capacitor, and a fuel cell.

In one embodiment, the gastric band **2010** can be, for example, a RAB with a fluid in the cushions. For example, the gastric band **2010** can be a RAB with inflatable compartments **2030** including fluids.

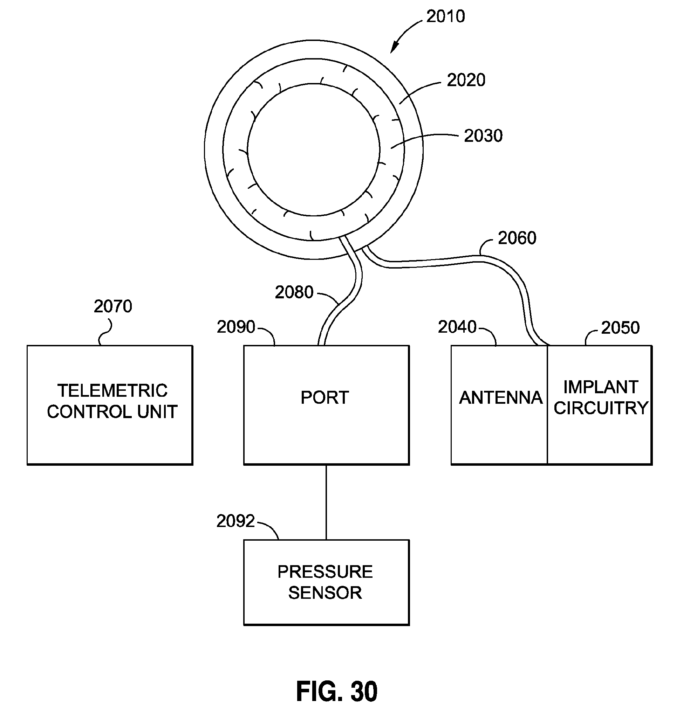

Turning now to FIG. 30, an implantable banding system in accordance with exemplary embodiments of the present invention optionally comprises an override mechanism. In exemplary embodiments, an override mechanism is configured to open the band in the event that there is an electrical failure, a mechanical failure, a power failure, a software failure, a hardware failure, or in the event that a telemetric control unit is not available. In an exemplary override mechanism, the fixed-volume compartment is attached to a port **2090** via tubing **2080** which is implanted in the body to allow for easy access to rapidly adjust the band open in the case of an emergency. The port **2090** can be, for example, a small low profile port. The port **2090** can be placed along side antenna **2040** for easy access.

An implantable banding system in accordance with exemplary embodiments of the present invention optionally comprises a pressure sensor **2092**. The pressure sensor **2092** can be connected to, or located within the port **2090**. Since the port **2090** can be in fluid communication with the gastric band **2010,** the pressure sensor **2092** can detect internal band pressure of the gastric band **2010** and can collect, for example pressure data. In addition, the pressure sensor **2902** can be located, for example, within the gastric band **2010,** such as on or within the inflatable compartment **2030,** or at an exterior of the inflatable compartment **2030.**

In exemplary embodiments, the implantable banding system comprises additional sensors in addition or instead of the pressure sensor **2092** positioned within the inner fluid compartment(s) configured to monitor a parameter of the fixed volume of fluid, optionally generate an adjustment signal based on the parameter and one or more parameter control limits, and optionally either automatically activate the mechanical adjustment mechanism based on the adjustment signal or transmit the adjustment signal to the telemetric control unit. In exemplary embodiments, the parameter may be selected from a pressure, a fill volume, a stress, a strain, a linear measurement, and/or combinations thereof. The data from the pressure sensor and/or other sensors can be transmitted, for example, to the implant circuitry **2050** and/or the telemetric control unit **2070.**

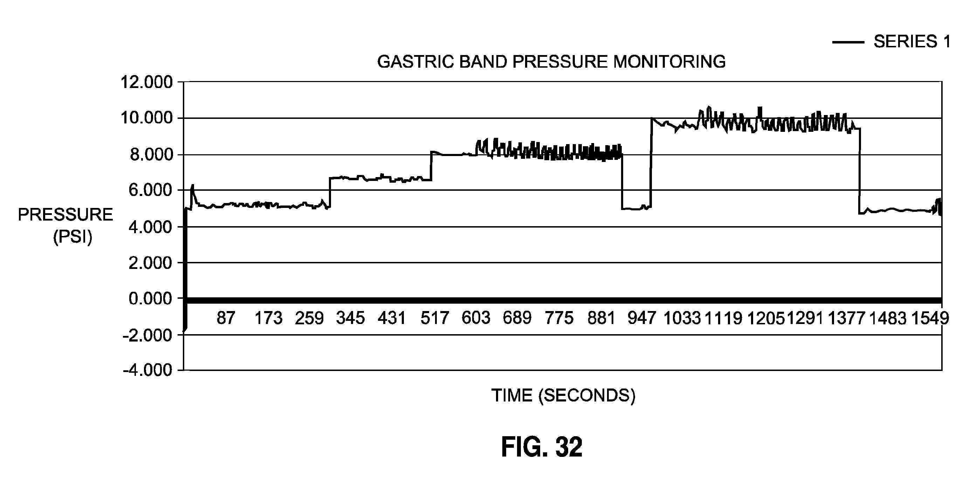

For example, the pressure sensor **2092** can collect pressure data as seen in FIG. 32. In FIG. 32, a needle can be placed in the port **2090** and connected to the pressure sensor **2092.** The pressure data can indicate when a patient was at a constriction that was inducing satiety, or when the gastric band **2010** was only slightly over-constricted. This can be seen by the very little variation in pressure between the time period 1 second and 517 seconds. The variation of the pressure can be, for example, less than 0.1 psi. When the gastric band was adjusted to the point where the gastric band was too constricted and the patient felt slightly uncomfortable, there was a greater variation in the pressure data as can be seen in the time period between 603 seconds and 861 seconds.

When the patient swallows water, gradual pressure curves could be seen. As the gastric band **2010** was increasingly constricted, the intra-band pressure response increased in variation as can be seen in the time period between 1033 seconds and 1377 seconds. In some situations, the pressure can vary, for example, by as much as 2 psi. This can provide, for example, the caregiver a new diagnostic tool to determine the optimum level of adjustment based on the pressure response in the gastric band **2010.**

Thus, the implant circuitry **2050** can analyze the pressure data from the pressure sensor **2092,** to determine whether to constrict or relax the gastric band **2010.** In one embodiment, the implant circuitry **2050** can incrementally constrict the gastric band **2010** a nominal constriction and analyze the pressure data for variation information such as standard deviation or greatest difference between maximum and minimum values. If the implant circuitry **2050** determines that the variation information such as the standard deviation is too great, then the implant circuitry **2050** can relax the gastric band by an incremental amount. Any adjustment to the gastric band **2010** can be stored as adjustment data by the implant circuitry **2050.** The adjustment data and/or the pressure data can be accessed, for example, by the telemetric control unit **2070** or other external device. The telemetric control unit **2070** can pass along the pressure data and/or the adjustment data to an external device, such as a caregiver's computer. The telemetric control unit **2070** and/or the caregiver's computer can see, for example, a graph such as that depicted in FIG. 32 indicating the pressure data. Thus, the gastric band **2010** can be fully self-automated and self-adjusting.

In one embodiment, the pressure data can be, for example, real-time data. In addition, the pressure data can correspond, for example, to the patient swallowing water and can indicate peristalsis. This can allow the caregiver to visualize the pressure curves generated inside the gastric band **2010** during the adjustment. In addition, average pressure, pressure standard deviation, pressure minimum and maximum measured over time can be collected and transferred to the telemetric control unit **2070** for display.

In one embodiment, the implant circuitry **2050** can be triggered by the telemetric control unit **2070** located at the caregiver's office. Once triggered, the implant circuitry **2050** will proceed to adjust the gastric band **2010** and allow, for example, the caregiver to observe the adjustment results.

Furthermore, the implant circuitry **2050** can also allow for an automatic adjustment of the gastric band **2010** when there is an obstruction. For example, the caregiver or the patient can trigger the implant circuitry **2050** using the telemetric control unit **2070.** The implant circuitry **2050** can detect an abnormally high pressure in the gastric band **2010** and to relax the gastric band **2010** to relieve pressure on the patient's stomach. After the obstruction has passed, the implant circuitry **2050** can be triggered again. The constriction of the gastric band **2050** can then be appropriately increased. If there is an ideal pressure for the gastric band **2010** for the specific patient, then the implant circuitry **2050** can also periodically monitor the pressure data to ensure that the gastric band **2010** is operating at the appropriate pressure.

In addition, the caregiver can also adjust the operating parameters of the gastric band **2010** and/or the implant circuitry **2050.** For example, the caregiver can increase or decrease the pressure threshold of the gastric band **2010.** In addition, the caregiver can increase or decrease the thresholds for the other data detected by the other sensors in the gastric band **2010.** For example, the implant circuitry **2050** may be monitoring between 2 psi and 3 psi when the caregiver queries or triggers the implant circuitry **2050** using, for example, the telemetric control unit **2070.** The caregiver can adjust the implant circuitry **2050** to monitor between 4.5 and 5.5 psi instead using the telemetric control unit **2070.** The implant circuitry **2050** can then increase or decrease the pressure of the gastric band **2010** accordingly so that it is within 4.5 and 5.5 psi. The implant circuitry **2050** can, for example, increase or decrease the amount of constriction by the gastric band **2010.**

For example, the implant circuitry will draw power from an implanted battery to allow for the adjustment and will also activate, for example, the check valves in the gastric band **2010** to open the check valves. To constrict the gastric band, the implant circuitry **2050** can instruct the stepper motor to constrict the gastric band **2010.** To relax the gastric band **2010,** the implant circuitry **2050** can instruct the stepper motor to relax the gastric band **2010.** Once the pressure sensor detects pressure data within the specified pressure range, such as 4.5 and 5.5 psi, the stepper motor will stop its constriction or relaxation. To confirm the new pressure data, the caregiver can use the telemetric control unit **2070** to query the implant circuitry **2050** for pressure data. The stepper motor and the pressure sensor can then be shut off until the telemetric control unit **2070** queries the implant circuitry **2050.**

The implant circuitry **2050** can also be programmed to wake up periodically and monitor the pressure data to readjust the gastric band **2010** as necessary to ensure that the pressure of the gastric band **2010** is still within the specified pressure range or other parameters. Any changes in the parameters or ranges for the pressure sensor or the other sensors in the gastric band **2010** can be permanently or semi-permanently recorded along with the date of the change and the delta of the change. This information can be supplied, for example, to the telemetric control unit **2070.** In addition, the stepper motor in the gastric band **2010** can be preprogrammed with a serial number that can be sent to the telemetric control unit **2070.**

In one embodiment, the telemetric control unit **2070** can include an LCD display and control panel to operate the telemetric control unit **2070.** In addition, the telemetric control unit **2070** can include a series of menus allowing a user to program the gastric band **2010** to include important information such as the gastric band size, patient's name, implanting physician, and the date that the gastric band **2010** was implanted.

In addition, the telemetric control unit **2070** can communicate with the gastric band **2010** and/or the implant circuitry **2050** using telemetry through radio waves. For example, the globally recognized communications bands WMTS 402 - 405 Mhz or 27 Mhz can be used. An authentication process can also be used to ensure that the gastric band **2010** cannot be accidentally accessed or controlled by another control mechanism aside from the telemetric control unit **2070.** The telemetric control unit **2070** can communicate with the gastric band **2010** and receive, for example, pressure data without requiring the patient to disrobe. The telemetric control unit **2070** can also be password controlled to prevent unauthorized personnel from using the device.

An exemplary pressure monitoring mechanism is described in U.S. Publication No. 2007/0156013, to the present inventor Birk. However, a pressure monitoring mechanism in accordance with the present invention should not be limited to what is disclosed in the foregoing publication. Instead, a pressure monitoring mechanism in accordance with the present invention should be broadly construed as any configuration designed or implemented to monitor the pressure exerted by the stomach on the fixed-volume compartment(s) or the mechanical adjustment mechanism **2020**.

An implantable banding system in accordance with exemplary embodiments of the present invention optionally comprises a self-adjusting mechanism, in turn comprising a sensor.

In accordance with exemplary embodiments, the sensor is a pressure sensor for obtaining a first pressure reading within the at least one inner fluid compartment at a first time and a second pressure reading within the at least one inner fluid compartment at a second time, and determining whether to automatically activate the mechanical adjustment mechanism based on the first pressure reading and the second pressure reading.

In accordance with other exemplary embodiments, the sensor is a pressure sensor for obtaining a minimum pressure reading within the at least one inner fluid compartment over a predetermined period of time and a maximum pressure reading within the at least one inner fluid compartment over the predetermined period of time, and determining whether to automatically activate the mechanical adjustment mechanism based on the minimum pressure reading and the maximum pressure reading. In accordance with one aspect of exemplary embodiments, the sensor calculates a standard deviation using the minimum pressure reading and the maximum pressure reading.

In accordance with yet other exemplary embodiments, the sensor is a linear motion sensor for determining a change in length of the gastric band between a first time and a second time, and determining whether to automatically activate the mechanical adjustment mechanism based on the change in the length of the band. In accordance with one aspect of exemplary embodiments, the sensor converts the length into a diameter.

An exemplary self-adjusting mechanism is described in U.S. Publication No. 2007/0156013. However, a self-adjusting mechanism in accordance with the present invention should not be limited to what is disclosed in the foregoing publication. Instead, a self-adjusting mechanism in accordance with the present invention should be broadly construed as any configuration designed or implemented to adjust the gastric band **2010** through a variety of diameters based on information received from the pressure monitoring mechanism.

An implantable banding system in accordance with exemplary embodiments of the present invention optionally comprises a drug delivery mechanism. In exemplary embodiments, a drug is injected into the override mechanism port **2090** for later release through the membrane of the inflatable compartment **2030**. In accordance with an aspect of an exemplary embodiment, the shell of the inflatable compartment **2030** is materially and/or structurally configured to optimize its function as a drug delivery membrane. In accordance with an aspect of another exemplary embodiment, the shell of the inflatable compartment **2030** is covered with a drug eluting coating for slow release of a bioactive agent.

In exemplary embodiments, the implantable banding system of the present invention is implanted by a conventional laparoscopic procedure. In exemplary embodiments, the physician first dissects the tissues around the stomach to create a tunnel for the gastric band **2010**. The gastric band **2010** is then introduced into the patient's abdomen, either through an 18mm trocar or directly through the trocar hole in the skin. The gastric band **2010** is then tunneled in place and positioned around the stomach. Finally, the antenna **2040** and the low profile port **2090** are placed just below the skin on top of the sternum.

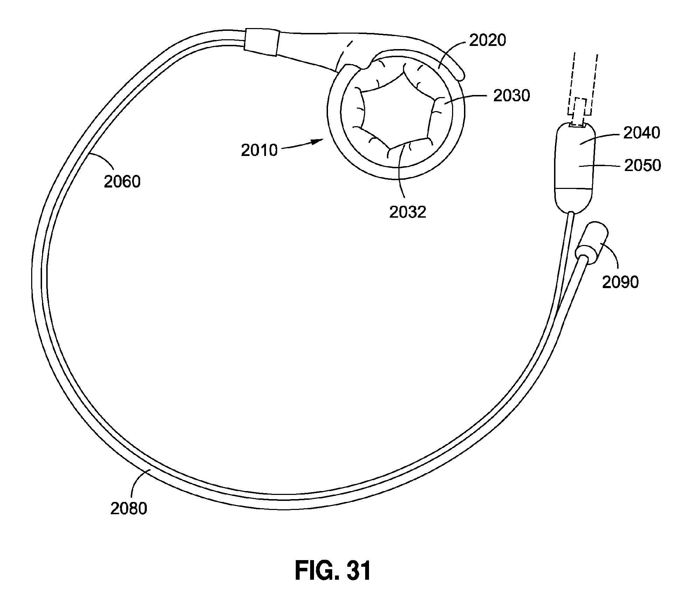

Turning finally to FIG. 31, a working embodiment of an implantable banding system in accordance with the present invention is shown.

As stated elsewhere herein, the system of the present invention has numerous applications apart from gastric banding. For example, the system of the present invention may be used for the treatment of fecal incontinence, ileostomy, coleostomy, gastro-esophageal reflux disease, urinary incontinence and isolated-organ perfusion.

For treatment of fecal incontinence, the ring may be used with little or no modifications. In addition, because the ring adjustment procedure will be performed by the patient on at least a daily basis, a portable user-friendly external control may be used. In addition, because the ring will regularly be transitioned between the closed and fully opened position, the patient microchip card is unneeded. Instead, the fully closed position may be stored in the memory of the implantable controller, and read by the external remote at each use (subject to periodic change by the physician).

A similarly modified device could be used by patients who have undergone ileostomy or coleostomy, or disposed surrounding the esophageal junction, to treat gastro-esophageal reflux disease.

For treatment of urinary incontinence, the system of the present invention may be further modified to minimize the volume of the loop surrounding the urethra by moving the actuator motor to a location elsewhere in the lower abdomen or pelvis, and coupling the actuator to the motor via a transmission cable.

The present invention also may be beneficially employed to perform isolated-organ perfusion. The treatment of certain cancers requires exposure to levels of chemotherapy agents that are too high for systemic circulation. It has been suggested that one solution to this problem is perform an open surgery procedure in which blood flow to the cancerous organ is stopped and quiescent blood replaced by circulation from an external source containing a desired dose of drug. Individual or multiple rings of the present invention may be used as valves to isolate the cancerous organ and permit perfusion of the organ with high doses of drugs. Such procedures could thus be performed on a repetitive basis without surgery, thereby reducing the trauma and the risk to the patient while improving patient outcomes.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or and consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. An implantable banding system for treating obesity, the implantable banding system comprising:

   a gastric band having at least one inner fluid compartment (2030) and an outer mechanical adjustment mechanism (2020), the at least one inner fluid compartment being filled with a volume of fluid, and the outer mechanical adjustment mechanism comprising a device configured to adjust the gastric band through a variety of diameters,
   wherein the volume of fluid in the at least one inner fluid compartment (2030) is a fixed volume of fluid.

2. The implantable banding system of claim 1 including a sensor (2092) positioned within the at least one inner fluid compartment (2030), configured to monitor a parameter of the fixed volume of fluid, and generate data based on the parameter to be monitored.

3. The implantable banding system of claim 2 including a telemetric control unit (2070), the telemetric control unit (2070) preferably being configured to receive data based on the parameter to be monitored, the telemetric control unit (2070) alternatively receiving a telemetric signal comprising data related to the gastric band.

4. The implantable banding system of claim 3 wherein the telemetric control unit (2070) further comprises a display for graphically displaying the parameter for a period of time, preferably additionally displaying the one or more parameter control limits for a period of time.

5. The implantable banding system of claim 3 wherein the telemetric control unit (2070) transmits a remote telemetric signal to the sensor, the remote telemetric signal comprising a request for the parameter and the one or more parameter control limits.

6. The implantable banding system of claim 3, 4 or 5 including an implant circuit (2050) coupled to the device and configured to analyze the data from the sensor (2092), and control operations of the device based on the data from the sensor (2092) including automatically activating the device based on the data from the sensor (2092 or transmit the data from the sensor to the telemetric control unit (2070).

7. The implantable banding system of claim 2 or 6 wherein the parameter is selected from a group consisting of a pressure, a fill volume, a stress, a strain, a linear measurement, and combinations thereof.

8. The implantable banding system of claim 2 or 6 wherein the sensor (2092) is a pressure sensor for obtaining a first pressure reading within the at least one inner fluid compartment at a first time and a second pressure reading within the at least one inner fluid compartment at a second time, and the implant circuit (2050) determines whether to automatically activate the mechanical adjustment mechanism based on the first pressure reading and the second pressure reading.

9. The implantable banding system of claim 2 or 6 wherein the sensor (2092) is a pressure sensor for obtaining a minimum pressure reading within the at least one inner fluid compartment over a predetermined period of time and a maximum pressure reading within the at least one inner fluid compartment over the predetermined period of time, and the implant circuit (2050) determines whether to automatically activate the mechanical adjustment mechanism based on the minimum pressure reading and the maximum pressure reading, the implant circuit preferably calculating a standard deviation using the minimum pressure reading and the maximum pressure reading.

10. The implantable banding system of claim 2 or 6 wherein the sensor is a linear motion sensor for determining a change in length of the gastric band between a first time and a second time, the sensor preferably converting the length into a diameter, and the implant circuit (2050) determines whether to automatically activate the mechanical adjustment mechanism based on the change in the length of the band.

11. The implantable banding system of claim 1 or 6 wherein the fluid is selected from a group consisting of a drug, a saline solution, and combinations thereof.

12. The implantable banding system of claim 1 or 6 wherein the device is selected from a group consisting of a stepper motor, the stepper motor preferably being configured to rotate around a flexible screw within the gastric band to adjust the gastric band through a variety of diameters, a pump, a valve, and combinations thereof.

13. The implantable banding system of claim 1 or 6 further comprising an override mechanism coupled to the inner fluid compartment for releasing the fluid contained therein in the event of a failure of the system, the failure preferably being selected from a group consisting of an electrical failure, a mechanical failure, a power failure, a software failure, a hardware failure, and combinations thereof.

14. The implantable banding system of claim 1 further comprising a power source selected from a group consisting of a battery, a capacitor, a fuel cell, and combinations thereof, the power source preferably being recharged by one or more of motion, a chemical change, and a temperature change.

**Patentansprüche**

1. Implantierbares Bandsystem zum Behandeln von Fettleibigkeit, wobei das implantierbare Bandsystem aufweist:

   ein Magenband, das mindestens einen inneren Fluidraum (2030) und einen äußeren mechanischen Einstellmechanismus (2020) aufweist, wobei der mindestens eine innere Fluidraum mit einem Fluidvolumen gefüllt ist und der äußere mechanische Einstellmechanismus eine Einrichtung aufweist, die eingerichtet ist, das Magenband über eine Vielfalt von Durchmessern einzustellen,
   wobei das Fluidvolumen in dem mindestens einen inneren Fluidraum (2030) ein festgelegtes Fluidvolumen ist.

2. Implantierbares Bandsystem nach Anspruch 1, einschließlich eines Sensors (2092), der in dem mindestens einen inneren Fluidraum (2030) positioniert und eingerichtet ist, einen Parameter des festgelegten Fluidvolumens zu überwachen und basierend auf dem zu überwachenden Parameter Daten zu erzeugen.

3. Implantierbares Bandsystem nach Anspruch 2, einschließlich einer telemetrischen Steuereinheit (2070), wobei die telemetrische Steuereinheit (2070) vorzugsweise eingerichtet ist, auf dem zu überwachenden Parameter basierende Daten zu empfangen, und die telemetrische Steuereinheit (2070) alternativ ein telemetrisches Signal empfängt, das mit dem Magenband in Zusammenhang stehende Daten aufweist.

4. Implantierbares Bandsystem nach Anspruch 3, bei dem die telemetrische Steuereinheit (2070) ferner eine Anzeige für ein grafisches Anzeigen des Parameters über einen Zeitraum und vorzugsweise zusätzliches Anzeigen der einen oder mehreren Parametersteuergrenzen über einen Zeitraum aufweist.

5. Implantierbares Bandsystem nach Anspruch 3, bei dem die telemetrische Steuereinheit (2070) ein telemetrisches Fernsignal an den Sensor überträgt, wobei das telemetrische Fernsignal eine Anfrage des Parameters und der einen oder den mehreren Parametersteuergrenzen aufweist.

6. Implantierbares Bandsystem nach Anspruch 3, 4 oder 5 einschließlich eines Implantatschaltkreises (2050), der mit der Einrichtung gekoppelt ist und eingerichtet ist, die Daten von dem Sensor (2092) zu analysieren, und Funktionen der Einrichtung basierend auf den Daten von dem Sensor (2092) einschließlich eines automatischen Aktivierens der Einrichtung basierend auf den Daten von dem Sensor (2092) zu steuern oder die Daten von dem Sensor an die telemetrische Steuereinheit (2070) zu übertragen.

7. Implantierbares Bandsystem nach Anspruch 2 oder 6, bei dem der Parameter aus einer Gruppe ausgewählt ist, die aus einem Druck, einem Füllvolumen, einer Spannung, einer Dehnung, einer Längenmessung und Kombinationen daraus besteht.

8. Implantierbares Bandsystem nach Anspruch 2 oder 6, bei dem der Sensor (2092) ein Drucksensor zum Ermitteln eines ersten Druckmesswerts in dem mindestens einen inneren Fluidraum zu einem ersten Zeitpunkt und eines zweiten Druckmesswerts in dem mindestens einen inneren Fluidraum zu einem zweiten Zeitpunkt ist und der Implantatschaltkreis (2050) bestimmt, ob der mechanische Einstellmechanismus basierend auf dem ersten Druckmesswert und dem zweiten Druckmesswert automatisch zu aktivieren ist.

9. Implantierbares Bandsystem nach Anspruch 2 oder 6, bei dem der Sensor (2092) ein Drucksensor zum Ermitteln eines minimalen Druckmesswerts in dem mindestens einen inneren Fluidraum über einen vorbestimmten Zeitraum und eines maximalen Druckmesswerts in dem mindestens einen inneren Fluidraum über den vorbestimmten Zeitraum ist und der Implantatschaltkreis (2050) bestimmt, ob das mechanische Einstellsystem basierend auf dem minimalen Druckmesswert und dem maximalen Druckmesswert automatisch zu aktivieren ist, wobei der Implantatschaltkreis vorzugsweise eine Standardabweichung unter Verwendung des minimalen Druckmesswerts und des maximalen Druckmesswerts berechnet.

10. Implantierbares Bandsystem nach Anspruch 2 oder 6, bei dem der Sensor ein Linearbewegungssensor zum Bestimmen einer Längenänderung des Magenbands zwischen einem ersten Zeitpunkt und einem zweiten Zeitpunkt ist, wobei der Sensor vorzugsweise die Länge in einen Durchmesser umwandelt und der Implantatschaltkreis (2050) bestimmt, ob der mechanische Einstellmechanismus basierend auf der Längenänderung des Bands automatisch zu aktivieren ist.

11. Implantierbares Bandsystem nach Anspruch 1 oder 6, bei dem das Fluid aus einer Gruppe ausgewählt ist, die aus einem Wirkstoff, einer Salzlösung und Kombinationen daraus besteht.

12. Implantierbares Bandsystem nach Anspruch 1 oder 6, bei dem die Einrichtung aus einer Gruppe ausgewählt ist, die aus einem Schrittmotor, wobei der Schrittmotor vorzugsweise eingerichtet ist, um eine flexible Schraube in dem Magenband zu drehen, um das Magenband über eine Vielfalt von Durchmessern einzustellen, einer Pumpe, einem Ventil und Kombinationen daraus besteht.

13. Implantierbares Bandsystem nach Anspruch 1 oder 6, ferner mit einem über Steuerungsmechanismus, der mit dem inneren Fluidraum zum Freigeben des darin enthaltenen Fluids in dem Fall eines Systemversagens gekoppelt ist, wobei das Versagen vorzugsweise aus einer Gruppe ausgewählt ist, die aus einem elektrischen Versagen, einem mechanischen Versagen, einem Stromausfall, einem Softwareversagen, einem Hardwareversagen und Kombinationen daraus besteht.

14. Implantierbares Bandsystem nach Anspruch 1, ferner mit einer Leistungsquelle, die aus einer Gruppe ausgewählt ist, die aus einer Batterie, einem Kondensator, einer Brennstoffzelle und Kombinationen daraus besteht, wobei die Leistungsquelle vorzugsweise durch eine Bewegung, eine chemische Änderung und/oder eine Temperaturänderung aufladbar ist.

**Revendications**

1. Système de cerclage implantable pour traiter l'obésité, le système de cerclage implantable comprenant :

un anneau gastrique ayant au moins un compartiment de fluide interne (2030) et un mécanisme d'ajustement mécanique externe (2020), l'au moins un compartiment de fluide interne étant rempli d'un volume de fluide, et le mécanisme d'ajustement mécanique externe comprenant un dispositif configuré pour ajuster l'anneau gastrique à travers une variété de diamètres,

dans lequel le volume de fluide dans l'au moins un compartiment de fluide interne (2030) est un volume fixe de fluide.

2. Système de cerclage implantable de la revendication 1, comportant un capteur (2092) positionné à l'intérieur de l'au moins un compartiment de fluide interne (2030), configuré pour surveiller un paramètre du volume fixe de fluide, et générer des données sur la base du paramètre à surveiller.

3. Système de cerclage implantable de la revendication 2, comportant une unité de commande télémétrique (2070), l'unité de commande télémétrique (2070) étant de préférence configurée pour recevoir des données sur la base du paramètre à surveiller, l'unité de commande télémétrique (2070) recevant alternativement un signal télémétrique comprenant des données liées à l'anneau gastrique.

4. Système de cerclage implantable de la revendication 3, dans lequel l'unité de commande télémétrique (2070) comprend en outre un dispositif d'affichage pour afficher graphiquement le paramètre pendant une période, de préférence pour afficher en outre la ou les plusieurs limite(s) de commande de paramètre pendant une période.

5. Système de cerclage implantable de la revendication 3, dans lequel l'unité de commande télémétrique (2070) transmet un signal télémétrique à distance au capteur, le signal télémétrique à distance comprenant une demande du paramètre et de la ou des plusieurs limite(s) de commande de paramètre.

6. Système de cerclage implantable de la revendication 3, 4 ou 5, comportant un circuit d'implant (2050) couplé au dispositif et configuré pour analyser les données provenant du capteur (2092), et commander des opérations du dispositif sur la base des données provenant du capteur (2092) comportant le fait d'activer automatiquement le dispositif sur la base des données provenant du capteur (2092) ou transmettre les données provenant du capteur à l'unité de commande télémétrique (2070).

7. Système de cerclage implantable de la revendication 2 ou 6, dans lequel le paramètre est choisi dans un groupe constitué d'une pression, d'un volume de remplissage, d'une contrainte, d'une tension, d'une mesure linéaire, et de combinaisons de ceux-ci.

8. Système de cerclage implantable de la revendication 2 ou 6, dans lequel le capteur (2092) est un capteur de pression pour obtenir une première lecture de pression à l'intérieur de l'au moins un compartiment de fluide interne à un premier moment et une deuxième lecture de pression à l'intérieur de l'au moins un compartiment de fluide interne à un deuxième moment, et le circuit d'implant (2050) détermine s'il faut activer automatiquement le mécanisme d'ajustement mécanique sur la base de la première lecture de pression et de la deuxième lecture de pression.

9. Système de cerclage implantable de la revendication 2 ou 6, dans lequel le capteur (2092) est un capteur de pression pour obtenir une lecture de pression minimale à l'intérieur de l'au moins un compartiment de fluide interne au cours d'une période prédéterminée et une lecture de pression maximale à l'intérieur de l'au moins un compartiment de fluide interne au cours de la période prédéterminée, et le circuit d'implant (2050) détermine s'il faut activer automatiquement le mécanisme d'ajustement mécanique sur la base de la lecture de pression minimale et de la lecture de pression maximale, le circuit d'implant calculant de préférence un écart-type en utilisant la lecture de pression minimale et la lecture de pression maximale.

10. Système de cerclage implantable de la revendication 2 ou 6, dans lequel le capteur est un capteur de mouvement linéaire pour déterminer une variation de longueur de l'anneau gastrique entre un premier moment et un deuxième moment, le capteur convertissant de préférence la longueur en un diamètre, et le circuit d'implant (2050) détermine s'il faut activer automatiquement le mécanisme d'ajustement mécanique sur la base de la variation de la longueur de l'anneau.

11. Système de cerclage implantable de la revendication 1 ou 6, dans lequel le fluide est choisi dans un groupe constitué d'un médicament, d'une solution saline, et de combinaisons de ceux-ci.

12. Système de cerclage implantable de la revendication 1 ou 6, dans lequel le dispositif est choisi dans un groupe constitué d'un moteur pas-à-pas, le moteur pas-à-pas étant de préférence configuré de manière à tourner autour d'une vis souple à l'intérieur de l'anneau gastrique pour ajuster l'anneau gastrique à travers une variété de diamètres, d'une pompe, d'une soupape, et de combinaisons de ceux-ci.

13. Système de cerclage implantable de la revendica-

**EP 2 582 334 B1**

tion 1 ou 6, comprenant en outre un mécanisme de neutralisation couplé au compartiment de fluide interne pour libérer le fluide contenu dedans en cas de panne du système, la panne étant de préférence choisie dans un groupe constitué d'une panne électrique, d'une panne mécanique, d'une panne de courant, d'une panne de logiciel, d'une panne de matériel, et de combinaisons de celles-ci.

14. Système de cerclage implantable de la revendication 1, comprenant en outre une source d'énergie choisie dans un groupe constitué d'une batterie, d'un condensateur, d'une pile à combustible, et de combinaisons de ceux-ci, la source d'énergie étant de préférence rechargée par l'un(e) ou plusieurs d'un mouvement, d'une variation chimique et d'une variation de température.

FIG. 1

CONTROL

16

10

18

14

15

24

30

22

28

20

26

44

17

12

EP 2 582 334 B1

FIG. 2

**FIG. 3**

**FIG. 3A**

**FIG. 4A**

**FIG. 4B**

EP 2 582 334 B1

**FIG. 4C**

FIG. 5A

FIG. 5B

FIG. 5D

FIG. 5C

FIG. 5F

FIG. 5E

FIG. 5H

FIG. 5G

EP 2 582 334 B1

FIG. 6A

FIG. 6B

FIG. 6C

31

**FIG. 7**

**FIG. 8**

**FIG. 9**

EP 2 582 334 B1

**FIG. 10**

**FIG. 11**

**FIG. 12**

**FIG. 13**

**FIG. 14**

**FIG. 15**

**FIG. 16A**

**FIG. 16B**

**FIG. 17**

**FIG. 18**

**FIG. 20**

**FIG. 21**

**FIG. 19**

**FIG. 22A**

**FIG. 22B**

**FIG. 22C**

**FIG. 22D**

**FIG. 22E**

**FIG. 22F**

**FIG. 22G**

**FIG. 22H**

**FIG. 23**

**FIG. 24**

**FIG. 25**

1033
52
60
44
60
26A

**FIG. 26**

1033
60
44
60
52

**FIG. 26A**

1033
44
60
52
27A

**FIG. 27**

1033
60
52
44

**FIG. 27A**

FIG. 28

**FIG. 29A**

**FIG. 29B**

**FIG. 30**

**FIG. 31**

GASTRIC BAND PRESSURE MONITORING

— SERIES 1

FIG. 32

2010

2020

2030

2033

**FIG. 33**

2010

2020

2033

2030

**FIG. 34**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5938669 A
- EP 1719480 A2
- US 2009270904 A1
- US 2007156013 A1
- US 2009054914 A1
- US 20050143766 A, Bachmann
- US 20050082793 A
- US 20050192531 A
- US 20070156013 A